(19) Europäisches Patentamt
European Patent Office
Office européen des brevets

(11) **EP 1 017 727 B1**

(12) **EUROPEAN PATENT SPECIFICATION**

(45) Date of publication and mention of the grant of the patent:
**20.08.2003 Bulletin 2003/34**

(51) Int Cl.[7]: **C08B 37/00**, A61K 35/78

(21) Application number: **98943613.4**

(86) International application number:
**PCT/CA98/00891**

(22) Date of filing: **23.09.1998**

(87) International publication number:
**WO 99/015566 (01.04.1999 Gazette 1999/13)**

(54) **COMPOSITION AND PHARMACEUTICAL PREPARATION CONTAINING SAME FOR THE TREATMENT OF HERPES AND RELATED VIRAL INFECTIONS**

ZUSAMMENSETZUNG UND DIESE ENTHALTENDE PHARMAZEUTISCHE ZUBEREITUNG FÜR DIE BEHANDLUNG VON HERPES UND VERWANDTEN VIRALEN INFEKTIONEN

COMPOSITION ET PREPARATION PHARMACEUTIQUE LA CONTENANT POUR LE TRAITEMENT DE L'HERPES ET AUTRES MALADIES VIRALES APPARENTEES

(84) Designated Contracting States:
**AT BE CH DE ES FR GB IT LI NL PT**

(30) Priority: **23.09.1997 US 59775 P**

(43) Date of publication of application:
**12.07.2000 Bulletin 2000/28**

(73) Proprietor: **DALHOUSIE UNIVERSITY**
**Halifax, Nova Scotia B3H 4H6 (CA)**

(72) Inventors:
• **LEE, Song, F.**
**Bedford, Nova Scotia B4A 3R6 (CA)**
• **LEE, Spencer, H., S.**
**Halifax, Nova Scotia B3H 1A4 (CA)**
• **XU, Hong-Xi**
**FO TAN, NT HONG KONG (HK)**
• **BLAY, Jonathan**
**Bedford, Nova Scotia B4A 4C5 (CA)**
• **FOONG, Wai-Choong**
**Bedford, Nova Scotia B4A 3R1 (CA)**

(74) Representative: **Maschio, Antonio**
**D Young & Co,**
**21 New Fetter Lane**
**London EC4A 1DA (GB)**

(56) References cited:
**WO-A-98/17282          US-A- 5 411 733**

• **PATENT ABSTRACTS OF JAPAN vol. 97, no. 12, 25 December 1997 & JP 09 202731 A (SEIKAGAKU KOGYO), 5 August 1997 & DATABASE WPI Week 9741 Derwent Publications Ltd., London, GB; AN 444056**
• **XIAO-JIAN YAO ET AL: "MECHANISM OF INHIBITION OF HIV-1 INFECTION IN VITRO BY PURIFIED EXTRACT OF PRUNELLA VULGARIS" VIROLOGY, vol. 187, no. 1, 1 March 1992, pages 56-62, XP000567722**
• **PATENT ABSTRACTS OF JAPAN vol. 96, no. 3, 29 March 1996 & JP 07 291815 A (MEIJI MILK PROD ), 7 November 1995 & DATABASE WPI Week 9602 Derwent Publications Ltd., London, GB; AN 017095**

**Description**

The present invention relates to a novel composition which can be extracted and purified from the spikes of *Prunella vulgaris.* Invention composition is an effective agent for treatment of the cytopathogenic effects of an enveloped virus in mammals. In another aspect, the present invention relates to methods for the treatment of the cytopathogenic effects of an enveloped virus and related indications in mammals, employing the invention composition as the active agent. In yet another aspect, the present invention relates to methods for the treatment of the cytopathogenic effects of an enveloped virus in mammals exposed to immunosuppressive regimens. In still another aspect, the present invention relates to methods for protecting mammals from reactivated viral infection during or following treatment with chemotherapeutic agents. In a further aspect, the present invention relates to methods for obtaining invention compositions having antiviral action from *Prunella vulgaris,* and formulations containing said compositions. In a still further aspect, the present invention relates to methods of purification of invention compositions from *Prunella vulgaris* and characterization thereof.

Viral infections caused by enveloped viruses are a heterogenous group of disorders characterized by viral infection of host cells. An example of such infections includes acquired herpes virus infections caused by broad categories of herpes-related viruses, including the alpha-, beta- and gamma-herpes viruses, and where the onset of infection is characterized by herpes simplex virus (HSV) infection of host cells. Primary herpes virus infections are normally acquired in childhood, but later enter a dormant phase (i.e., in the nerves). Reactivation of herpes virus infections result from a variety of factors, such as ultraviolet light, stress, and adult onset.

Extensive work is being done to identify compounds that inhibit the pathogenicity of viruses and to prevent or treat viral infections. While some anti-viral agents are presently available, there is a great need for additional compounds that would be more active, and more specific in preventing or treating various viral infections.

The incidence and severity of herpes virus infections have increased over the last decade as a result of disease pattern, the frequency of drug use and the emergence of drug and analog resistant herpes infections, resulting in new interest in the pathophysiology of HSV enveloped virus diseases and their progression to identify more active and more specific compounds to prevent or treat HSV infections.

Herpes virus related infections are ubiquitous. Approximately 16-35%, 40-80%, and over 90% of the United States population by their third decade of life are seropositive for or infected by herpes simplex virus type 1 (HSV-1), herpes simplex virus type 2 (HSV-2), and varicella zoster virus (VZV), respectively. HSV-1 is the cause of cold-sores, keratitis, and encephalitis. HSV-2 is responsible for genital herpes and VZV is the causative agent for chicken pox and shingles. Five other members of the herpes family, specifically the Epstein-Barr virus (infectious mononucleosis and Burkitt's lymphoma), cytomegalovirus (congenital CMV infections), human herpes virus 6 and 7 (fever and rash in children), and human herpes virus 8 (Kaposi's Sarcoma), also cause disease in humans.

Currently, two classes of antiviral drugs are utilized clinically against herpes infections. The first class is represented by nucleoside analogs such as acyclovir and its prodrug derivatives (e.g. valaciclovir and famciclovir) and adenine arabinoside (ara-A). Acyclovir inhibits viral DNA synthesis selectively in HSV-infected cells. Acyclovir is activated by the viral thymidine kinase and is phosphorylated to acyclovir-triphosphate which is incorporated into the DNA chain by the viral DNA polymerase.

The second class of antiviral drugs are direct HSV DNA polymerase inhibitors, such as phosphonoformate (Foscovir) and phosphonoacetate. Resistance to these drugs arises from mutation in the thymidine kinase and in the DNA polymerase.

Over the past decade, the incidence and severity of herpes infections have increased due to the increase in the number of immunocompromised patients produced by aggressive chemotherapy regimens, expanded organ transplantation, and the rising incidence of human immunodeficiency virus (HIV) infection. This change in disease pattern and the increase in frequency of drug use have resulted in the emergence of acyclovir- and other nucleoside analog-resistant herpes infections, and a need for new and useful antiviral agents, especially those with a different mode of action than acyclovir.

The inhibitory effects of polyanionic substances on the replication of herpes simplex virus (HSV) and other viruses were reported almost four decades ago. However, these observations did not generate much interest, because the antiviral action of the compounds was considered to be largely nonspecific. Shortly after the identification of human immunodeficiency virus (HIV) as the causative agent of the acquired immune deficiency syndrome (AIDS) in 1984, heparin and other anionic (sulfated) polysaccharides were found to be potent inhibitors of HIV-1 replication in cell culture. Since 1988, the activity spectrum of the anionic polysaccharides has been shown to extend to various enveloped viruses, including viruses that emerge as opportunistic pathogens (e.g., herpes simplex virus (HSV) and cytomegalovirus (CMV)) in immunosuppressed (e.g., AIDS) patients. As potential anti-viral drug candidates, anionic polysaccharides offer a number of promising features.

Accordingly, there is still a need in the art for effective compounds and methods for the prevention and treatment of the cytopathogenic effects caused by each of the various forms of viral infections caused by enveloped viruses.

In accordance with the present invention, we have discovered that a novel composition which can be extracted and purified from the spikes of *Prunella vulgaris* is an effective agent for the treatment of the cytopathogenic effects of an enveloped virus in mammals. In accordance with another embodiment of the invention, we have discovered methods for treatment of the cytopathogenic effects of an enveloped virus and related indications in mammals, employing the purified compositions of the invention as the active agent. In accordance with yet another embodiment of the invention, we have discovered that the purified compositions of the invention are effective agents for the treatment of the cytopathogenic effects of an enveloped virus in mammals exposed to immunosuppressive regimens. In accordance with still another embodiment of the invention, we have discovered that the purified compositions of the invention are effective agents for protecting mammals from reactivated viral infection during or following treatment with chemotherapeutic agents. In accordance with a further embodiment of the invention, we have discovered methods for obtaining invention compositions having antiviral action from *Prunella vulgaris;* in addition, there are also provided formulations containing said compositions. In accordance with a still further embodiment of the invention, we have discovered methods of purification of invention compositions from *Prunella vulgaris,* and characterization thereof.

BRIEF DESCRIPTION OF THE FIGURES

Figure 1 presents a representative method for extraction and isolation of an active anti-herpes extract from the spikes of *Prunella vulgaris.*

Figure 2 presents a representative separation of active anti-herpes materials by a Sephadex G-50 column.

Figure 3 collectively presents the HPLC analysis of anti-herpes preparations from *Prunella vulgaris.* Thus, Figure 3A presents the HPLC elution profile of an aqueous extract; Figure 3B presents the HPLC elution profile of a partially purified extract (PVP; see Example 1), and Figure 3C presents the HPLC elution profile of Fraction E (see Example 2).

Figure 4 illustrates the lack of cytotoxicity of the *Prunella vulgaris* aqueous extracts up to 500 µg/ml.

Figure 5 illustrates the further separation of active anti-herpes materials by a BioGel P4 column.

Figure 6 presents HPLC profile of the purified *Prunella vulgaris* polysaccharide.

Figure 7 presents a graph presenting an estimation of the molecular mass of the *Prunella vulgaris* polysaccharide by HPLC.

In accordance with the present invention, there are provided compositions in substantially purified form, said compositions characterized as:

(1) water soluble polyanionic polysaccharide(s) comprising glucose, galactose and xylose, wherein glucose is the major constituent, and galactose and xylose are minor components as analysed by paper chromatography;
(2) having substantially little or no anti-coagulant activity as measured by the prothrombin time test;
(3) being able to inhibit viral infection before virus binding as well as after virus binding and penetration;
(4) having substantially little or no *in vivo* toxicity; and
(5) having a molecular mass of approximately 3300 Da. Invention compositions can be further characterized as:
(6) having a pH of 5.5 when dispersed in an aqueous solution at a concentration of about 1 mg/ml;
(7) having a strong UV absorption peak at 202 nm and a shoulder at 280 nm extending to 380 nm when dispersed in aqueous medium;
(8) being stable to exposure to temperatures in the range of about 95-100°C for 4 hours;
(9) being substantially insoluble in methanol, ethanol, butanol, acetone, and chloroform;
(10) having an elemental content of about 30.78% carbon, 3.05% hydrogen, 0.66% nitrogen and 2.69% sulfur;
(11) containing 42% (w/w) carbohydrate, expressed as gluouronic acid;
(12) containihg 7.5% (w/w) uronic acid, expressed as glucuronic acid;
(13) capable of binding to Alcian blue and to DEAE Sepharose at neutral pH;
(14) being non-proteinaceous ;
(15) having a retention time of 3.56 min when subjected to reversed-phase high pressure liquid chromatography (HPLC) on a C18 column (25cmx4.6mmID, 5µ, Supelcosil LC-18, Sigma) and eluted with 5% water : 95% acetonitrile at a flow rate of 0.3 ml/min; and

(16) being effective for the treatment of the cytopathogenic effects of an enveloped virus in a mammal.

The novel compositions of the present invention can be prepared by a variety of established methods for both extraction and purification. One such method is the one described in Example 1 and Example 2 in the present specification. In another approach, for example; invention compositions can be obtained from cells of the plant *Prunella vulgaris,* by purifying the invention composition by contacting an extract from *Prunella vulgaris* with an anion exchange material which selectively binds negatively charged materials, and recovering the invention composition from the anion exchange material.

The present invention is also directed to pharmaceutical formulations suitable for the treatment of the cytopathogenic effects of an enveloped virus in a mammal in need thereof, which contains an effective amount of invention composition, with or without an appropriate pharmaceutically acceptable carrier therefor. Examples of enveloped virus include Herpes simplex virus type 1 and 2, human immunodeficiency virus type 1 (HIV-1), human cytomegalovirus, measles virus, mumps virus, influenza and parainfluenza virus, and respiratory syncytial virus.

The present invention is also directed to pharmaceutical formulations suitable for the treatment of cytopathogenic effects of an enveloped virus, more preferably herpes simplex virus, in a mammal in need thereof, which formulation contains an effective amount of said purified composition or an effective amount of said purified composition together with a pharmaceutically acceptable carrier therefor.

The present invention is also directed to methods for treating the cytopathogenic effects of an enveloped virus which comprises administering to a mammal in need thereof an effective amount of the above-described pharmaceutical formulation, or of said purified composition, optionally with a pharmaceutically acceptable carrier. More particularly, the mammal to be treated is a human who has been diagnosed as having an infection caused by said enveloped virus. Most particularly the mammal to be treated is a human who has been specifically diagnosed as having herpes simplex virus.

In accordance with another embodiment of the present invention, there are provided methods for the treatment of the cytopathogenic effects of an enveloped virus in a mammal. Invention methods comprise administering to a mammal in need thereof an effective amount of the above-described purified composition.

As used herein, "mammal" refers to humans as well as other mammals, and includes animals of economic importance such as bovine, ovine, and porcine animals. The preferred mammal contemplated for treatment according to the invention is a human. Adults as well as non-adults (i.e., neo-nates, pre-pubescent mammals, and the like) are contemplated for treatment in accordance with the invention.

As used herein, the phrase "cytopathogenic effect of an enveloped virus" refers to the abnormal condition of a cell caused by the infection by an enveloped virus. As noted above, viral infections caused by enveloped viruses are a heterogenous group of disorders characterized by viral infection in host cells. Viral infections caused by enveloped viruses can be acquired as a direct result of exposure to an enveloped virus, or reactivation of an enveloped virus infection in a seropositive mammal, and which reactivation may be induced by exposure to a broad category of agents, including but not limited to a variety of environmental factors (e.g., stress), immunocompromising regimens (i.e., pertaining to an immune response that has been weakened by a disease or a chemotherapy agent or an immunosuppressive agent), weakened immunocompetence arising from other viral infections (such as HIV and the like), age onset, and the like. As described herein, disorders characterized by viral infections in cells include infections of cultured cells (i.e., lytic infections, persistent infections, latent infections, transforming infections, abortive infections, and the like), infectious disorders, conditions and diseases (i.e., acute infections, inapparent or silent infections, chronic and persistent infections, latent infections, slowly progressive diseases, virus-induced tumours, and the like), and the like.

The compositions of the present invention are preferably present in a purified form when administered to a patient. When invention compositions are obtained by extraction from plant spikes, it is desirable to separate soluble extract from (residual) particulate matter by appropriate means (e.g., filtration, centrifugation, or other suitable separation techniques). The utility of invention compositions as a therapeutic agent is enhanced by greater purification. Greater dosages may be necessary when less pure forms of the extract are employed.

Invention compositions are preferably substantially free from heavy metals, contaminating plant materials, contaminating microorganisms, oxalic acid or precursors of oxalic acid or any other contaminants which may be present in a preparation which can be derived from plant material.

Invention compositions can also be used to inhibit the cytopathogenic effects of enveloped viruses (e.g., HSV) in mammals in need thereof, more particularly in humans.

Although isolation of invention compositions from the spikes of *Prunella vulgaris* plants is the presently most practical method for obtaining such materials, the present invention also contemplates obtaining such materials from other sources such as species within the subfamily Nepetoideae, of which *Prunella* is a member. It is also possible that invention compositions could be obtained by culturing plant cells, such as *Prunella vulgaris* cells, *in vitro* and extracting the active ingredients from the cells or recovering the active ingredients from the cell culture medium.

As used herein, the term "extract" means the active ingredients isolated from spikes or other parts of *Prunella vulgaris* or other natural sources including but not limited to all varieties, species, hybrids or genera of the plant regardless of the exact structure of the active ingredients, form or method of preparation or method of isolation. The term "extract" also intended to encompass salts, complexes and/or derivatives of the extract which possess the above-described biological characteristics or therapeutic indication. The term "extract" is also intended to cover synthetically or biologically produced analogs and homologs with the same or similar characteristics yielding the same or similar biological effects of the present invention.

The purified compositions contemplated for use herein include purified extract fractions having the properties described herein from any plant or species preferably *Prunella vulgaris*, in natural or in variant form, and from any source, whether natural, synthetic, or recombinant. Also included within the scope of the present invention are analogs and homologs of the above-described purified compositions.

The present invention also contemplates the use of synthetic preparations having the characteristics of invention compositions. Such synthetic preparations could be prepared based on the chemical structure and/or functional properties of the above-described compositions of the present invention. Also contemplated are analogs and homologs of the chemical structure of the invention compositions and having the functional properties of compositions according to the present invention.

As used herein, reference to "analogs and homologs" of the invention compositions embraces compounds which differ from the structure of invention compositions by as little as the addition and/or replacement and/or deletion of one or more residues thereof, to compounds which have no apparent structural similarity. Such compounds in all instances, however, have substantially the same activity as invention compositions. Thus, "analogs" refers to compounds having the same basic structure as invention compositions, but differing in several residues; "homologs" refers to compounds which differ from invention compositions by the addition and/or deletion and/or replacement of a limited number of residues.

As used herein, "treatment" refers to therapeutic and prophylactic treatment. Those in need of treatment include those already with enveloped virus infections as well as those in which treatment of the enveloped virus infection has failed.

Invention compositions described for use herein can be delivered in a suitable vehicle, thereby rendering such composition amenable to oral deliver, transdermal delivery, subcutaneous delivery (e.g., intravenous delivery, intramuscular delivery, intraarterial delivery, intraperitoneal delivery, and the like), topical delivery, inhalation delivery, osmotic pump, and the like. Depending on the mode of delivery employed, the above-described composition can be delivered in a variety of pharmaceutically acceptable forms. For example, the above-described composition can be delivered in the form of a solid, solution, emulsion, dispersion, micelle, liposome, and the like.

Pharmaceutical formulations contemplated for use in the practice of the present invention contain invention composition in admixture with an organic or inorganic carrier or excipient suitable for enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used include glucose, lactose, gum acacia, gelatin, mannicol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening and coloring agents and perfumes may be used. The active compounds contemplated for use herein are included in the pharmaceutical formulation in an amount sufficient to produce the desired effect upon the target enveloped viral disease.

Pharmaceutical formulations containing the active compound contemplated herein may be in a form suitable for oral use, for example, as tablets, troches, lozenges, aqueous or oily suspensions, dispersible powders or granules, emulsions, hard or soft capsules, or syrups or elixirs. Formulations intended for oral use may be prepared according to any method known in the art for the manufacture of pharmaceutical formulation. In addition, such formulations may contain one or more agents selected from a sweetening agent (such as sucrose, lactose, or saccharin), flavoring agents (such as peppermint, oil of wintergreen or cherry), colouring agents and preserving agents, and the like, in order to provide pharmaceutically elegant and palatable preparations. Tablets containing the active components in admixture with non-toxic pharmaceutically acceptable excipients may also be manufactured by known methods. The excipients used may be, for example, (1) inert diluents such as calcium carbonate, lactose, calcium phosphate, sodium phosphate, and the like; (2) granulating and disintegrating agents such as corn starch, potato starch, alginic acid, and the like; (3) binding agents such as gum tragacanth, corn starch, gelatin, acacia, and the like; and (4) lubricating agents such as magnesium stearate, stearic acid, talc, and the like. The tablets may be uncoated or they may be coated by known techniques to delay disintegration and absorption in the gastrointestinal tract, thereby providing sustained action over a longer period. For example, a time delay material such as glyceryl monostearate or glyceryl distrearate may be employed. They may also be coated by the techniques described in the U.S. Pat. Nos. 4,256,108; 4,160,452; and 4,265, 874, to form osmotic therapeutic tablets for controlled release.

In some cases, formulations for oral use may be in the form of hard gelatin capsules wherein the active components are mixed with an inert solid diluent, for example, calcium carbonate, calcium phosphate, kaolin, or the like. They may also be in the form of soft gelatin capsules wherein the active components are mixed with water or an oil medium, for example, peanut oil, liquid paraffin, or olive oil.

The pharmaceutical formulation may be in the form of a sterile injectable suspension. This suspension may be formulated according to known methods using suitable dispersing or wetting agents and suspending agents. The sterile injectable preparation may also be a sterile injectable solution or suspension in a non-toxic parenterally-acceptable diluent or solvent, for example, as a solution in 1,3-butanediol. Sterile, fixed oils are conventionally employed as a solvent or suspending medium. For this purpose any bland fixed oil may be employed including synthetic mono- or diglycerides, fatty acids (including oleic acid), naturally occurring vegetable oils like sesame oil, coconut oil, peanut oil, cottonseed oil, etc., or synthetic fatty vehicles like ethyl oleate or the like. Buffers, preservatives, antioxidants, and the like can be incorporated as required.

It may be desirable to administer in conjunction with the invention composition other viral DNA synthesis inhibitors or DNA polymerase inhibitors that promote synergistic therapeutic and prophylactic effects.

The treatment regimen or pattern of administration of the agents may be one with simultaneous administration of an agent which counteracts the effects of invention compositions, and invention compositions. In addition, the treatment regimen may be phasic with an alternating pattern of administration of one agent followed at a later time by the administration of the second agent. Phasic administration includes multiple administrations of one agent followed by multiple administrations of the second agent. The sequence that the agents are administered in and the lengths of each period of administration would be as deemed appropriate by the practitioner.

Invention compositions can also be suitably administered employing sustained-release systems. Suitable examples of sustained-release compositions include semi-permeable polymer matrices in the form of shaped articles, e.g. films, or microcapsules. Sustained-release matrices include polylactides (U.S. Pat. No. 3,773,919), copolymers of L-glutamic acid and gamma-ethyl-L-glutamate (Sidman *et al*., Biopolymers, 22:547-556(1983)), poly (2-hydroxyethyl-methacrylate) (Longer *et al*., J. Biomed, Mater, Res., 15:267-277 (1981)), ethylene vinyl acetate (Langer *et al., supra)* or poly-D-(-)-3 hydroxybutyric acid (EP133,988), and the like. Sustained-release formulations containing invention compositions also include liposomally entrapped compositions according to the invention. Liposomes are prepared by methods known in the art (see, for example, DE 3,218,121; U.S. Pat. Nos. 4,485,045 and 4,545,545).

For parenteral administration, in one embodiment, invention compositions are formulated by mixing in a unit dosage injectable form (solution, suspension, or emulsion), with a pharmaceutically acceptable carrier, i.e., one that is non-toxic to recipients at the dosages and concentrations employed and is compatible with other ingredients of the formulation. The formulation preferably does not include oxidizing agents and other compounds that are known to be deleterious to polypeptides.

Generally, the formulations are prepared by contacting invention compositions uniformly and intimately with liquid carriers or finely divided solid carriers or both. Then, if necessary, the product is shaped into the desired form. Preferably the carrier is a parenteral carrier, more preferably a solution that is isotonic with the blood of the recipient. Examples include water, saline, Ringers solution, dextrose solution, and the like. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as poly-vinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxmers, or PEG. Invention compositions are typically formulated in such vehicles according to clinically relevant/acceptable protocol. It will be understood that use of certain of the foregoing excipients, carriers, or stabilizers will result in the formation of salts of invention compositions.

In addition, invention compositions are suitably formulated in an acceptable carrier vehicle to form a pharmaceutical formulation, preferably one that does not contain cells. In one embodiment, the buffer used for formulation will depend on whether the resulting formulation will be employed immediately upon mixing or stored for later use. If employed immediately, invention compositions can be formulated in mannitol, glycine, and phosphate at an appropriate pH. If this mixture is to be stored, it is preferably formulated in a buffer at an appropriate pH, in the optional further presence of a surfactant that increases the solubility of invention compositions at this pH. The final preparation may be a stable liquid or a lyophilized solid.

While invention compositions can be formulated in any way suitable for administration, presently preferred formulations contain about 2-20 mg/mL of invention composition, about 2-50 mg/mL of an osmolyte, about 1-15 mg/mL of a stabilizer, and a buffered solution at about pH 5-6, more preferably pH about 5-5.5. Preferably, the osmolyte is an inorganic salt at a concentration of about 2-10 mg/mL or a sugar alcohol at a concentration of about 40-50 mg/mL, the stabilizer is benzyl alcohol or phenol, or both, and the buffered solution is an acetic acid salt buffered solution. Even more prefered are formulations wherein the osmolyte is sodium chloride and the acetic acid salt is sodium acetate. Still more preferably, the amount of invention composition is about 8-12 mg/mL, the amount of sodium chloride is about 5-6 mg/mL, the amount of benzyl alcohol is about 8-10 mg/mL, the amount of phenol is about 2-3 mg/mL, and the amount of sodium acetate is about 50 mM so that the pH is about 5.4. Additionally, the formulation can contain about 1-5 mg/mL of a surfactant, preferably polysorbate or poloxamer, in an amount of about 1-3 mg/mL. Alternatively, for the preparation of invention formulation, invention composition is suitably dissolved at 5 mg/ml in 10 mM citrate buffer and 126 mM NaCI at pH 6.

Invention compositions to be used for therapeutic use must be sterile. Sterility is readily accomplished by filtration through sterile filtration membranes (e.g., 0.2 micron membranes). Therapeutic compositions and formulations according to the invention generally are placed into a container having a sterile access port, for example, a vial having a stopper pierceable by a hypodermic injection needle.

Invention compositions and formulations ordinarily will be stored in unit or multi-dose containers, for example, sealed ampules or vials, as an aqueous solution, or as a lyophilized formulation for reconstitution. As an example of a lyophilized formulation, 10-ml vials are filled with 5 ml of sterile-filtered aqueous solution of composition according to the invention, and the resulting mixture is lyophilized. The infusion solution is prepared by reconstituting the lyophilized material in bacteriostatic Water-for-Injection.

Typical daily doses of the active component, in general, lie within the range of from about 10 µg up to about 1 g per kg body weight, and, preferably within the range of from about 100 µg up to about 500 mg per kg body weight and can be administered up to five times daily, for example. Presently preferred daily doses lie within the range of from about 1 mg up to about 50 mg per kg body weight. Typically, the active compound will be present in an amount of 0.1 to 90 percent by weight of the pharmaceutical formulation, preferably 0.5 to 50 percent by weight of the pharmaceutical formulation, for example. Where one will operate within the above ranges will vary depending on the route of administration and on a variety of considerations, such as, for example, the age of the patient, the size of the patient, other dysfunctions of the patient, what, if any, other medications the patient may be taking, and the like.

As a general proposition, the total pharmaceutically effective amount of invention composition administered parenterally per dose will be an amount sufficient to provide a therapeutic effect without inducing a significant level of toxicity. Since individual subjects may present a wide variation in severity of symptoms and each active ingredient has its unique therapeutic characteristics, it is up to the practitioner to determine a subject's response to treatment and vary the dosages accordingly.

Based on the *in vitro* data presented herein, a concentration of 10 µg to 50 mg of invention composition (see Example 4) per gram of cream or ointment is expected to be effective for topical application in preventing the cytopathogenicity of the herpes simplex virus.

In accordance with another embodiment of the present invention, there are provided methods for the treatment of the cytopathogenic effects of an enveloped virus and related indications in mammals in need thereof, said method comprising administering an effective amount of invention composition to said mammal.

Patients who present the cytopathogenic effects of an enveloped virus contemplated for treatment in accordance with the present invention are those testing seropositive to an enveloped virus, preferably, those who are diagnosed with HSV, VZV or HIV infections.

In accordance with yet another embodiment of the present invention, there are provided methods for the treatment of the cytopathogenic effects of an enveloped virus in mammals exposed to immunosuppressive regimens, said method comprising administering an effective amount of invention composition to said mammal.

Patients who present the cytopathogenic effects of an enveloped virus and exposed to immunosuppressive regimens contemplated for treatment in accordance with the present invention are those whose normal immunotolerance is compromised by exposure to immunosuppressive agents following, for example, organ transplantation, xenotransplantation, subjects who have undergone chemotherapy (e.g., cancer patients), and the like.

In accordance with still another embodiment of the present invention, there are provided methods for protecting mammals exposed to or following treatment with chemotherapeutic agents, from the cytopathogenic effects of a reactivated enveloped virus infection, said method comprising administering an effective amount of invention composition to said mammal.

Patients for whom protection from the reactivated cytopathogenic effects of an enveloped virus during or following treatment to chemotherapeutic agents is indicated include patients suffering from any disease which is commonly treated by the administration of chemotherapeutic agents, e.g., post organ transplant, nephrotic syndrome, cancer patients, and the like.

In accordance with a further embodiment of the present invention, there are provided methods for preparing novel extract fractions of *Prunella vulgaris* having antiviral action and formulations containing said extract fractions.

See, for example, the methods described in Examples 1, 2 and 3 provided herein.

The extract or extract fractions that are active in preventing the cytopathogenic effects of an enveloped virus could be used to treat the cytopathogenic effects of enveloped viral infections in mammals, most preferably humans, in the following way:

(i) Since the untreated aqueous extract has been administered to mammalian cells up to concentrations as high as 0.5 mg/ml (see Example 8) with no apparent side effects, it could also be ingested by human subjects and may reduce the potential for viral associated disease.

(ii) The compound that is active against the enveloped virus could also be administered intravenously to patients with viral infection after the compound has been further purified. The compound may be more effective when administered I.V. than orally since it is unlikely that 100% ofthe active compound would be absorbed from the gastrointestinal tract.

(iii) It is also conceivable that the purified compound active against the enveloped virus could be administered into the spinal fluid and may prevent the potential for enveloped viral associated disease in patients with an enveloped viral infection.

It is contemplated that the extract will be formulated into a pharmaceutical composition comprising an effective amount of the extract with or without a pharmaceutically acceptable carrier (as previously described). All references and patents cited herein are hereby incorporated by reference.

A number of natural products are known to have inhibitory effects on herpes simplex virus. Musci and Pragai (see Experientia 41:6 (1985)) showed the inhibitory effect of four flavonoids, i.e., quercetin, quercitrin, rutin, and hesperidine, on HSV-1 and Suid (alpha) HSV-1 (pseudorabies virus). A direct relationship between viral inhibition and the ability of flavonoids to increase cyclic AMP in the host cells was observed, suggesting flavonoids exert their antiviral effects via cyclic nucleotide metabolism. Wleklik et al. (see Acta Virol. 32:522 (1988)) further showed that hydroxylation at positions 3,5,7,3', and 4' of flavonoids was associated with the highest anti-herpes activity. Hayashi et al. (see Antimicrob. Agents Chemother. 36:1890-1893 (1992)) described inhibition of HSV-1 and HSV-2 replication in Vero cells by a bifavanone ginkgetin isolated from *Cepalotaxus drupacea*. The $IC_{50}$ (50% inhibition concentration) against HSV-1 was 0:91 μg/ml. Ginkgetin suppressed viral protein synthesis and had no effect on the binding and penetration of HSV-1 into cells. Barnard et al. (see Chemother. 39:203-211(1993)) described a flavonoid polymer of 2100 Dathat inhibited HSV penetration into cells.

Polysaccharides are known to affect the growth of animal viruses (see Shannan, W.M. in G.J. Galasso, T.C. Merigan, and R.A. Buchanon (ed.), Antiviral agents and viral diseases of man. Raven Press, New York (1984) at p. 55-121). In particular, anionic polysaccharides, such as heparin, dextran sulfate, carrageenans, pentosan polysulfate, fucoidan, and sulfated xylogalactans, are potent inhibitors of herpes virus binding to host cells (see, for example, Gonalez et al. in Antimicrob. Agents Chemother. 31:1388-1393 (1987); Baba et al. in Antimicrob. Agents Chemother. 32:1742-1745 (1988); and Damonte et al. Chemother. 42:57-60 (1996)). The activity spectrum of the sulfated polysaccharides has been shown to extend to various enveloped viruses (e.g., human immunodeficiency virus type 1 (HIV-1), measles virus, mumps virus, influenza and parainfluenza virus, respiratory syncytial virus (HSV) and cytomegalovirus (CMV)), including viruses that emerge as opportunistic pathogens. These polysaccharides are competitor of receptors (heparan sulfate) to viral glycoproteins. Herold et al. (see J. Virol. 70:3461-3469 (1996)) showed that N-sulfations and the presence of carboxy groups on heparin are key determinants for HSV-1 and HSV-2 interactions with host cells. However, these polysaccharides also have anti-coagulant activity and are therefore unsuitable as anti-herpes drugs.

Some plant proteins are known to have anti-herpes activities. The better known ones are ribosome-inactivating proteins (e.g. pokeweed anti-viral protein (see, for example, Teltow et al. in Antimicrob. Agents Chermother. 23:390 (1983)) and lectins (e.g. concanavalin A; see, for example, Okada, Y., and J. Kim. Virology 5:507 (1972)). Pokeweed anti-viral protein is a 30 kDa single polypeptide that binds irreversibly to HSV and enters host cells only after binding to the virus. Following entry into the host cells, the protein inhibits protein synthesis. Concanavalin A interacts with the viral envelope to inhibit infectivity or to block exit of virus from infected cells. More recently, the anti-HSV activity of two other plant proteins and one human serum protein were described. MAP30 and GAP31 are a 30 kDa and 31 kDa protein isolated from the Chinese bitter melon *Momordica charantia* and a Himalayan tree *Gelonium multiflorum*, respectively (see, for example, Bourinbaiar, A.S., and S. Lee-Huang, in Biochem. Biophys. Res. Comm. 219:923-929 (1996)). Both proteins showed $IC_{50}$ against HSV-1 and HSV-2 in the 0.1 to 0.5 μM range. The mode of action of MAP30 and GAP31 is not known. The high density serum apolipoprotein A-1 was found to inhibit HSV-induced cell fusion at 1 μM (see Srinivas, R.V., et al. in Virology 176:48-57 (1990)). An 18 amino acid synthetic peptide analog to apolipoprotein A-1 inhibited penetration of virus into cells but did not prevent virus adsorption.

Other anti-herpes natural products include terpenoids and tannins. Terpenoids (e.g. glycyrrhizic acid, see, for example, Vanden Berghe, D.A., et al., in Bull. Inst. Pasteur 84:101 (1986)) inhibit HSV replication (see Hudson, J.B. in Antiviral compounds from plants. CRC Press, Inc. Boca Raton, FL (1990)). Tannins are thought to inhibited viral adsorption (see, for example, Fukudri, K., et al. in Antiviral Res. 11:285-297 (1989)). Xu et al. (see Heterocycles 38: 167-175 (1994)) showed that a hydrolyzable tannin, geponin, and gallic aldehyde had $IC_{50}$ against HSV-1 of 25 and 12.5 μg/ml, respectively.

*Prunella vulgaris,* a perennial plant commonly found in China, the British Isles, and Europe, has been used as an astringent for internal and external purposes (see, for example, Grieve, M. in A modern herbal. Dover Publications, NY. (1973)), as a crude anticancer drug (see, for example, Lee, H., and J.Y. Lin. in Mutation Res. 204:229-234 (1988)), and as a herbal remedy to lower high blood pressure (see, for example, Namba, T. in The encyclopedia of Wakan-Yaku (traditional Sino-Japanese medicines), Vol II, p. 120-121; Hoikusha Publishing Co. Ltd. Osaka, Japan (1994)). In western herbal remedies, the plant (which is better known as "self heal") is used in the form of hot water infusion sweetened with honey to treat sores in the mouth and throat (see, for example, Grieve, M. in A modern herbal. Dover Publications, NY. (1973)). Zheng (see Chung-Hsi-I-Chieh-Ho-Tsa-Chih. 10:39-41 (1990)) reported the use of a crude aqueous extract of *Prunella* vulgaris in clinical treatment of herpetic keratitis with some success. Of the 78 patients who received eye drops containing crude extracts of *Prunella vulgaris* and *Pyrrosia lingua,* 38 were reported to be cured, 37 showed improvement, and 3 did not respond. A crude aqueous extract of *Prunella vulgaris* contained no detectable anti-coagulant activity (see Zeng, F.-Q. M.Sc. Thesis. National University of Singapore (1996)). Hence, while there is some evidence that *Prunella vulgaris* is an anti-herpes plant, the active anti-herpes components are not known.

HSV-1, HSV-2, and VSV belong to the human alpha-herpes viruses, while cytomegalovirus and Epstein-Barr virus belong to the beta-herpes and gamma-herpes virus, respectively. HSV are large (180 - 200 nm in diameter) enveloped viruses containing double stranded DNA. The DNA core is surrounded by an icosahedral capsid containing 162 capsomers. The capsid is in turn, enclosed by a glycoprotein-containing envelope, composed of at least 11 known glycoproteins (gB, gC, gD, gE, gG, gH, gI, gJ, gK, gL, and gM), a number of which are responsible for viral attachment to cells, and the fusion of infected cells. Between the envelope and the capsid is the tegument, which contains other viral proteins.

HSV infection of host cells is a multi-step event beginning with binding of the virion to cell surface proteoglycans. Heparin sulfate moieties on proteoglycans are the site of virus binding, although HSV can also bind to chondroitin sulfate. Two viral glycoproteins, gC and gB, are responsible for HSV attachment to heparin sulfate. gC is the principal player in the binding and when it is absent from the virion, gB mediates binding at a reduced efficiency. A subsequent step in HSV entry into the cell involves the interaction of gD with a second cell surface molecule, possibly the mannose-6-phosphate receptor (see Brunetti, C.R., et al. in J. Biol. Chem 269:17067-17074 (1994)). HSV penetration occurs by fusion of the viral envelope with the cell membrane. This fusion is pH-independent and requires the participation of at least four viral glycoproteins, gB, gD, gH, and gL. Following the fusion, viral nucleocapsid is released into the cytoplasm and is uncoated to allow the viral DNA to enter the nucleus. Viral multiplication occurs in the nucleus in an orderly fashion with the initial appearance of immediate-early proteins necessary for regulation of gene transcription, early proteins (e.g. DNA polymerase), and late proteins (structural proteins). The progeny virions are widely believed to be assembled in the nucleus and exit from the cell through the endoplasmic reticulum.

In accordance with the present invention, extracts from more than 20 plants have been screened for anti-HSV-1 activity using the standard plaque reduction assay (see Edgar, L., et al. in Manual of clinical microbiology-5th ed. A. Balows (chief ed.) American society for Microbiology, Washington DC (1991), p. 1184-1191). The hot-water extract prepared from the spike of *Prunella vulgaris* showed good activity and was not cytotoxic. A partially purified extract (PVP) was prepared from the freeze-dried aqueous extract by ethanol precipitation. The anti-herpes extract was further purified by gel permeation column chromatography (Sephadex G-50). One fraction (Fraction E) with anti-herpes activity was collected. HPLC analysis using a reversed-phase (ODS-2) column showed that fraction E contained one major peak and two very minor peaks. Plaque reduction assay showed that PVP and Fraction E had an $IC_{50}$ against HSV-1 of 18 and 10 μg/ml, respectively.

The purified extract was also active on clinical isolates and acyclovir-resistant (thymidine kinase-deficient and DNA polymerase-deficient) strains of HSV-1 and HSV-2. Preincubation of HSV-1 with the purified extract abolished the infectivity of the virus; however, pretreatment of Vero cells did not prevent HSV-1 infection, confirming that the extract prevented the early event (binding and/or penetration) of infection. In a one-step growth study, addition of PVP at 0, 2, 4, and 7.25 h after infection showed a 99, 96, 94 and 90% reduction in total (extracellular and intracellular) viral yield, respectively. These results confirm that the compound also interfered with viral replication.

Fraction E contained 42% (w/w) carbohydrate (expressed as glucuronic acid) as determined by the phenol sulfuric acid assay (see Dubois, M., et al. 1956. Anal. Biochem. 28:350-356 (1956)). It contained only 7.5% (w/w) uronic acid (expressed as glucuronic acid) as determined by the uronic acid assay described by Blumenkrantz and Asboe-Hansen (see Blumenkrantz, N., and G. Asboe-Hansen in Anal. Biocehm. 54:484-489(1973). The compound is polyanionic, as evidenced from its binding to Alcian blue (Whiteman, P. in Biochem. J. 131:343-350 (1973)) and to DEAE Sepharose at neutral pH. Hexosamines and proteins were not detected. As analyzed by paper chromatography, the purified anti-herpes polysaccharide was identified to be composed of glucose, galactose and xylose.

The purified extract was water soluble, but was insoluble in methanol, ethanol, butanol, acetone, or chloroform. The aqueous solution (1 mg/ml) of Fraction E had a pH of 5.5. Spectrophotometry showed a strong absorption peak at 202 nm and a shoulder at 280 nm which extended to 380 nm. The molecular mass of the purified compound, estimated by HPLC with a gel filtration column, was 3,500 Da. The polyanionic polysaccharide, prunellin, previously isolated by Tabba et al. (see Antiviral Res. 11:263-274(1989)) had a pH of 7.4 in aqueous solution, showed an adsorption peak at 370 nm which extended to 500 nm, and has a molecular mass of 10,000 Da. This confirms that the anti-herpes compound of Fraction E is also a polyanionic carbohydrate, and is chemically different from prunellin.

In accordance with the present invention, it has been discovered that extracts of *Prunella vulgaris* are effective in the treatment of anti-herpes viruses. The plant is known to contain oleanolic acid, triterpene acids (ursolic acid), triterpenoids, flavonoids (rutin), fenchone, tannins, and prunellin (see, for example, Namba, T. in The encyclopedia of Wakan-Yaku (traditional Sino-Japanese medicines), Vol II, p. 120-121; Hoikusha Publishing Co. Ltd. Osaka, Japan (1994)). Prunellin is a 10 kDa anionic polysaccharide and it has been shown to inhibit the replication of human immunodeficiency virus-1 (see, for example, Tabba, H. D., in Antiviral Res. 11:263-274 (1989), and Yao, X.-J., et al. in Virology 187:56-62 (1992)). Rutin has been shown to have some activity against HSV (see, for example, Mucsi, I., and B.M. Pragai. in Experientia 41:6(1985)), however, none of the other compounds have been demonstrated to have activity against herpes viruses.

The laboratory rabbit eye and mouse ear infection models are well characterized as experimental models in HSV infections. These and other animal infection models can be used to study the utility of the present extract fraction from *Prunella vulgaris* in preclinical evaluations.

The examples that follow describe extraction of *Prunella vulgaris* plant spikes, characteristics of the extract and the effect of the extract on inhibition of the cytopathogenic effects of herpes simplex virus.

The results described in the examples clearly show that the *P. vulgaris* polysaccharide is a more superior antiviral agent than known anti-herpes. In contrast with heparin, a known anti-coagulant, PVP had an average prothrombin time similar to that of water, indicating that PVP has no or substantially no anti-coagulant activity. In addition, *P. vulgaris* anionic polysaccharide was found to inhibit HSV infection before virus binding as well as after virus binding and penetration. The one-step growth study (Example 6) clearly show that even after 7.25 h post virus infection, the *P. vulgaris* polysaccharide could reduce virus yield by 90%. This is in real contrast to sodium heparin which has anti-herpes activity only when it is present at the start of the virus infection, i.e., only when the virus has not yet bound to the cells to initiate the infection cycle.

The following examples are illustrative only and are not intended to limit the scope of the invention as defined by the appended claims. Thus, it is intended that the present invention cover the modifications and variations of this invention provided they come within the scope of the appended claims and their equivalents.

Example 1

Extraction of Invention Composition from *Prunella vulgaris*

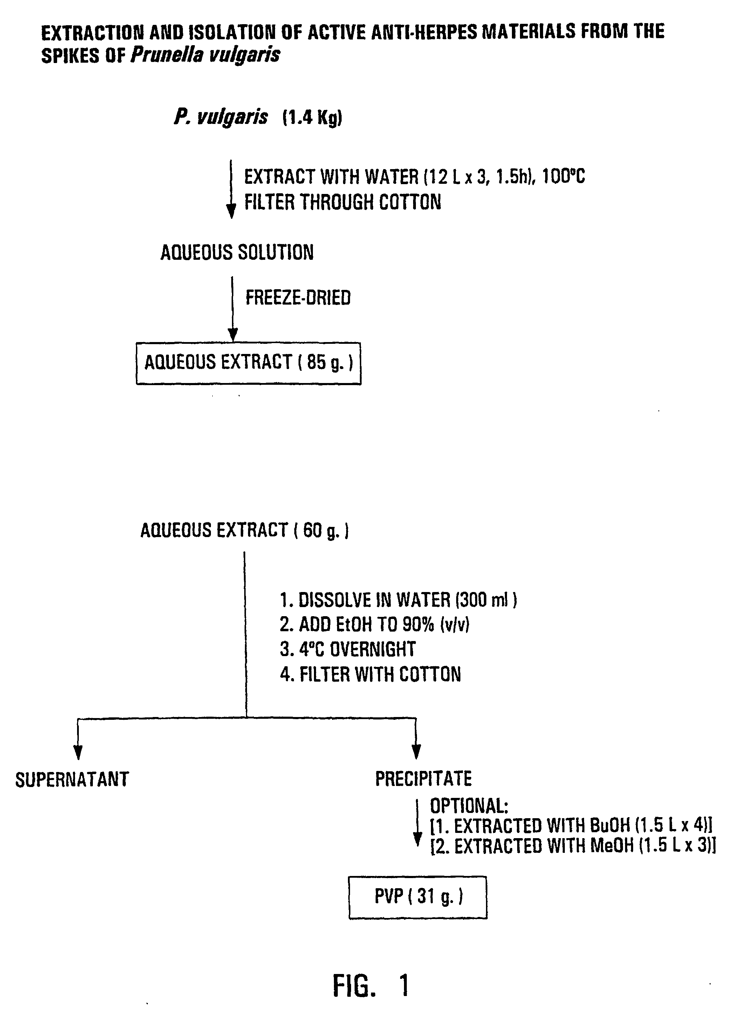

Dried spikes of *Prunella vulgaris* (1.4 kg) were ground into small pieces with a Waring blender. Distilled water (12 L) was added and the suspension was simmered at 95 - 100°C for 90 min. The extract was decanted to a clean container and the plant was extracted two more times with water under the same conditions. The extracts were poured through a cotton cloth to remove insoluble plant materials. The volume of the clarified extracts was reduced to about 1 litre by a rotary evaporator. The condensed extract was freeze-dried. A total of 85 g of dark brown dried powder was obtained (see Fig. 1).

The anti-herpes component in the aqueous extract was precipitated by ethanol. To achieve this, 30 g of the freeze-dried aqueous extract was dissolved in 300 ml of water, and ethanol was added to a final concentration of 90 % (vol/vol). The mixture was incubated at 4°C for 18 h. The precipitate was recovered by filtration through cotton and washed with 4 x 1.5 L of butanol, followed by 3 x 1.5 L of methanol. This yielded 31 g of dark brown powder, designated PVP (see Figure 1). The supernatant from ethanol precipitation contained no detectable anti-herpes activity by the plaque reduction assay (see Example 2) and was discarded.

Example 2

Purification of Extract Fraction by Column Chromatography

The active anti-herpes component was further purified by gel filtration column chromatography. An aqueous solution of PVP (450 mg in 10 ml) was applied to a Sephadex G-50 column (98 x 2.5 cm) and eluted with water.

Fractions of 5 ml were collected. The anti-herpes activity was detected using the plaque reduction assay. To do this, fractions were freeze-dried and redissolved in distilled water to 1 mg/ml. Plaque reduction assay was performed according to the standard method described by Edgar et al. (see Manual of clinical microbiology-5th ed. A. Balows (chief ed.) Amer. Soc. for Microbiology, Washington DC. (1991), p. 1184-1191). Briefly, monolayers of Vero cells grown on culture plates were infected with 100-200 pfu (plaque-forming unit) of virus. After incubation for 1 h to allow viral adsorption, the inoculum was aspirated and overlaid with medium (Dulbecco's Modified Eagle's medium with 2% fetal calf serum) containing dilutions of the *Prunella vulgaris* extract in 2% methylcellulose. After 72 h of incubation at 37 °C, the plates were fixed with formalin, stained with crystal violet, air dried and the number of plaques counted. Plates overlaid with medium without the extract were used as controls. The percentage of inhibition of plaque formation was calculated as:

$$\frac{(\text{\# of plaques in control}) - (\text{\# of plaques in test})}{(\text{\# of plaques in control})]} \times 100$$

Active fractions were pooled and freeze-dried. The control plates in which test compound was omitted had an average of 188.5 plaques/well.

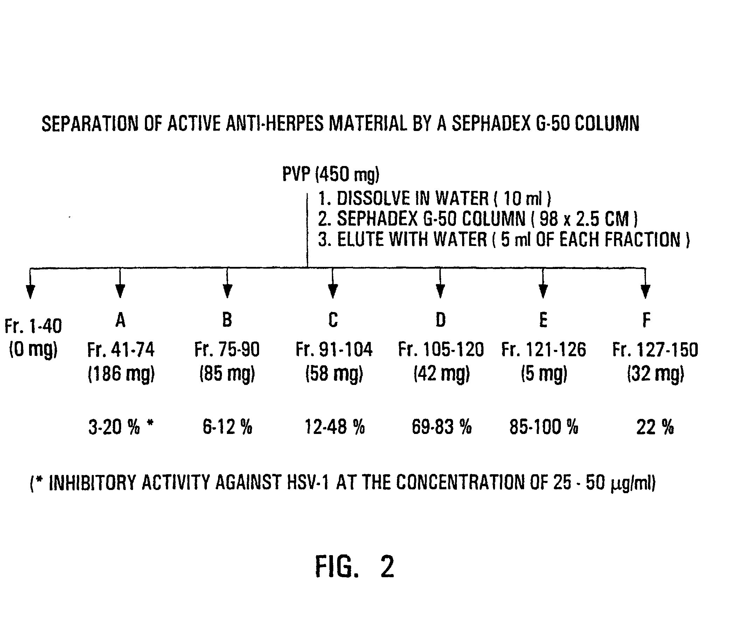

The active anti-herpes activity was found in fractions number 91 to number 131 (see Table 1, which presents the anti-HSV-1 activity of fractions from the Sephadex G-50 column). These fractions were pooled to give pooled fractions C, D, E, and F with the highest activity found in fraction E (see Figure 2). The amount of material recovered in each of the pooled fractions was indicated.

Table 1
Anti-HSV-1 activity of fractions from the Sephadex G-50 column

| Pooled fractions | Fraction No. | % Plaque inhibition | | |
|---|---|---|---|---|
| | | 75 µg/ml | 50 µg/ml | 25 µg/ml |
| A (186 mg) | 41 | NT[a] | 0 | 0 |
| | 45 | 0 | 0 | 0 |
| | 51 | 17.8 | 7.2 | 2.9 |
| | 56 | 22.0 | 11.9 | 0 |
| | 61 | 2.4 | 0 | NT |
| | 65 | 11.5 | 2.4 | NT |
| | 71 | 26.8 | 0 | NT |
| B (85 mg) | 75 | 49.1 | 16.2 | 0 |
| | 81 | 79.8 | 56.5 | 21.5 |
| | 85 | 86.7 | 46.9 | 10.3 |
| C (58 mg) | 91 | 100 | 49.6 | 7.7 |
| | 95 | 100 | 100 | 11.9 |
| | 101 | 100 | 100 | 45.9 |
| D (42 mg) | 105 | 100 | 100 | 65.5 |
| | 111 | 100 | 100 | 92.0 |
| | 115 | 100 | 100 | 82.5 |
| E (5 mg) | 121 | 100 | 100 | 84.6 |
| | 125 | 100 | 100 | 100 |
| F (32 mg) | 131 | 100 | 54.9 | 19.4 |
| PVP | | 100 | 73.5 | 36.3 |

[a] NT = not tested

The results of this example confirm that the purified extract fraction isolated from *Prunella vulgaris* of the present invention has anti-herpes activity.

Example 3

HPLC Analysis of the Purified Extract Fraction

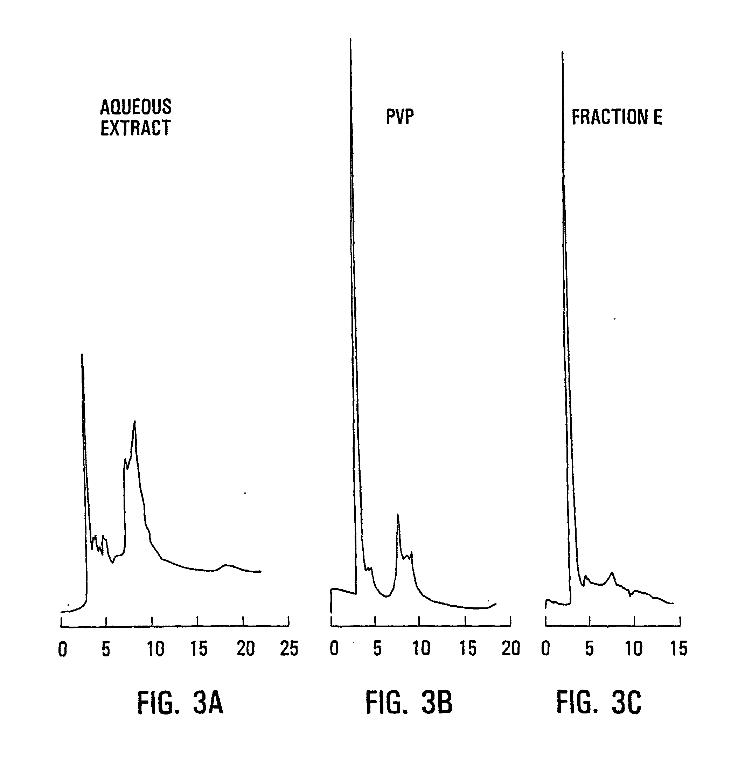

Purity of the anti-herpes materials from *Prunella vulgaris* was assessed by analyzing three preparations (aqueous extract, PVP, and Fraction E) by reversed-phase high pressure liquid chromatography (HPLC). Aliquots (25 µl) of aqueous solutions (10 µg/ml) were injected into a C18 column (25 cm x 4.6 mm ID, 5µ, Supelcosil LC-18, Sigma). Compounds were eluted with 5% water: 95% acetonitrile at a flow rate of 0.3 ml/min. Compounds were detected with a UV detector at 210 nm. The peak with a retention time of 3.56 min was concentrated during the purification process (see Figure 3). In Fraction E, this 3.56 min peak was essentially the only peak. Together with the anti-herpes test results provided in Example 4 (which demonstrate an increase in specific anti-HSV-1 activity during purification), the results of this example confirm that the 3.56 min peak is the purified extract fraction of *Prunella vulgaris,* which purified extract fraction has enhanced anti-herpes activity.

Example 4

Inhibitory Activity, IC$_{50}$

Potency of the anti-herpes compound from *Prunella vulgaris* was assessed by using a range of concentrations of PVP and Fraction E was used to inhibit plaque formation by HSV-1 in Vero cells. The percentage of plaque inhibition was dependent on the amount of PVP and Fraction E added (see Table 2). IC$_{50}$ is defined as the concentration of extract that causes 50% inhibition in the number of plaques formed in the assay system described. Zero inhibition is where the number of plaques formed, during the assay, is equivalent to or more than the number of plaques formed by control, where the extract is not present. One hundred percent inhibition is where there are no plaques formed during the assay. The concentration of PVP and Fraction E required to give 50% inhibition (IC$_{50}$) was calculated to be 18 and 10 µg/ml, respectively. Table 2 presents dose dependent inhibition of plaque formation by PVP and Fraction E (the control which contained no test compound had an average of 64.25 plaques/well).

Table 2

| Dose dependent inhibition of plaque formation by PVP and fraction E | | | |
|---|---|---|---|
| PVP (µg/ml) | % plaque inhibition | Fraction E (µg/ml) | % plaque inhibition |
| 50 | 96.9 | 50 | 100 |
| 25 | 61.1 | 25 | 100 |
| 12.5 | 35.4 | 12.5 | 55.6 |
| 6.25 | 29.2 | 6.25 | 35.4 |
| 3.125 | 0 | 3.125 | 13.6 |

The results of this example confirm the effectiveness and utility of the purified extract fraction from *Prunella vulgaris* against the herpes simplex virus.

Example 5

Spectrum of Activity

A number of viruses were used in the plaque reduction assay to assess the spectrum of activity of the extract fraction from *Prunella vulgaris.* Results showed that PVP at 100 µg/ml provided complete inhibition of plaque formation in Vero cells by laboratory and clinical strains of HSV-1 and HSV-2. PVP, at 100 µg/ml, also inhibited acyclovir-resistant strains of HSV-1 [strain DM2-1 (thymidine kinase-deficient) and strain PAAr5 (DNA polymerase-deficient)] and HSV-2 strain Kost (thymidine kinase altered). PVP at 100 µg/ml showed no activity against cytomegalovirus, human influenza virus types A and B, poliovirus type 1, and vesicular stomatitis virus. For the test with cytomegalovirus and human influenza viruses, human foreskin cells and MDCK (dog kidney) cells, respectively, were used in place of Vero cells.
The results ofthis example confirm that the extract fraction from *Prunella vulgaris* has specific activity against HSV-1 and HSV-2 and further demonstrates that since it is active against acyclovir-resistant HSV, the extract fraction from *Prunella vulgaris* can be a useful drug of choice in treating the cytopathogenic effects of infections caused by acyclovir-resistant HSV.

Example 6

Effects of *Prunella vulgaris* Anti-Herpes Compound on HSV-1 Infection

The mode of action of the anti-herpes effect of PVP was studied. Vero cells were preincubated with 75 µg/ml PVP for 16 to 20 h at 37°C. Cells were washed with medium and infected with HSV-1. The same number of plaques (50 plaques/well) was observed as that in controls in which cells had not been treated with PVP. This indicates that preincubation of Vero cells with PVP has no protective effect.

To study whether preincubation of virus with PVP will abolish infectivity, 100 µg of PVP was incubated with $10^5$ pfu of HSV-1 at 37°C. After 1 h incubation, the mixture was diluted 10,000 fold with medium and used to infect Vero cells. No plaque was observed on the monolayers after incubation. In contrast, the control plate, in which the viruses were pretreated with medium instead of PVP, contained an average of 130 plaques/well. This finding indicates that the infectivity of HSV-1 was abolished by preincubation with PVP, which probably exerts its effect by binding to the viral particles and preventing them to bind to heparan sulfate on Vero cells. This mode of action to reduce infectivity of herpes is completely different from acyclovir and other known nucleoside analogs.

The protective effect of PVP was demonstrated by adding PVP simultaneously with HSV-1 to Vero cells. When 75 µg/ml of PVP was added at the same time with HSV-1 to Vero cells, greater than 98% reduction in plaque formation was observed. Incubation of 100 µg PVP and $10^6$ plaque-forming units of HSV at 4°C, ambient temperature (25°C) and 36°C for 1 h abrogated 99% of the virus infectivity. The protective effect of PVP was further demonstrated when PVP was added post infection.

The effect of PVP on HSV-1 growth in Vero cells was further investigated in a one-step growth study. Monolayers of Vero cells were infected, at 4°C, with HSV-1 at a multiplicity of infection (MOI) of 5, i.e. 5 virions per cell. At this temperature, the virus would bind but would not penetrate the cells. Hence, all cells in the monoiayer were synchronized at the same step of viral infection. The cells were washed with cold medium and treated with 75 µg/ml PVP at 0, 2, 4, and 7.25 h after the washing step. Total viral yield at each time point was determined by plating out samples from the supernatant (extracellular) and lyzed cells (intracellular). Results confirm that when PVP was added at 0, 2, 4, and 7.25 h after infection, the total viral yield was reduced by 99, 96, 94, and 90%, respectively, as compared to controls where cells were not treated with PVP.

The results of this example confirm that the extract fraction from *Prunella vulgaris* also acts on herpes virus intracellularly to reduce the yield of infectious virus and prevents cell-to-cell transmission of the virus, and that the extract fraction interferes intracellularly with certain biosynthetic steps in the viral replication process.

Example 7

Chemical Nature of Anti-Herpes Compound from *Prunella vulgaris*

The chemical nature of the anti-herpes compound was investigated by different chemical tests. Total carbohydrate content was estimated by the phenol sulfuric acid assay using glucuronic acid as the standard, which results showed the anti-herpes compound in Fraction E contained 42% (w/w) carbohydrates (expressed as glucuronic acid). Uronic acids were measured with the method described by Blumenkrantz and Asboe-Hansen (see Anal. Biocehm. 54: 484-489 (1973)) using glucuronic acid as the standard. The anti-herpes compound contains 7.5% (w/w) uronic acid (expressed as glucuronic acid). Total hexosamines were determined by the Molgan-Elson reagent (see Whiteman, P. in Biochem. J. 131:343-350 (1973)) using N-acetylglucosamine (Sigma) as the standard. Hexosamines were not detected. Protein was measured by the Coomassie Blue dye binding method (Bio-Rad) using bovine serum albumin as the standard. Protein has not been detected. Elemental analysis showed the purified extract fraction to contain 30.78% carbon, 3.05% hydrogen, 0.66% nitrogen and 2.69% sulfur.

The anti-herpes compound was found to be precipitated by the cationic dye Alcian blue 8GX according to the assay method described by Whiteman (see Biochem. J. 131:343-350 (1973)). The anti-herpes compound bound strongly to DEAE Sepharose at neutral pH and could be eluted with 2 M NaCl. These experiments confirm the anti-herpes extract fraction from *Prunella vulgaris* is a polyanionic carbohydrate.

The anti-herpes compound was water soluble, but was insoluble in methanol, ethanol, butanol, acetone, or chloroform. The compound is heat stable (95-100°C, 4h). A 1 mg/ml aqueous solution of the anti-herpes compound gave a pH of 5.5. Spectrophotometry showed a strong absorption peak at 202 nm and a shoulder at 280 nm which extended to 380 nm. In contrast, the prunellin previously isolated by Tabba et al (see Antiviral Res. 11:263-274 (1989)) has a pH of 7.4 in aqueous solution, and an absorption peak at 370 nm which extended to 500 nm.

The results of this example confirm that the anti-herpes extract fraction from *Prunella vulgaris* is different from prunellin.

Example 8

Cytotoxicity

The cytotoxic effect of the aqueous extract on mammalian cells was tested using a rat intestinal epithelial cell line (RIE-1) according to a published method (see Blay, J., and A.S.L. Poon in Toxicon. 33:739-746 (1995)). Other cell lines including those originating from humans, such as T84 human intestinal epithelioid cells and KB human oral epidermoid cells can also be tested to ensure the lack of toxicity is not limited to one cell line or species. The dried aqueous extract was dissolved in DMSO and diluted in culture medium (Dulbecco's Modified Eagle's medium with 5% (v/v) heat-inactivated calf serum) before added directly to RIE-1 cells. The cultures were incubated for 48 h. MTT (3-[4,5-dimethylthiazol-2-yl]-2,5-diphenyl tetrazolium bromide) was added to the culture wells to give a final concentration of 0.5 mg/ml. The cultures were incubated for 3 h at 37°C to allow the conversion of MTT to formazan dye by mitochondrial succinate dehydrogenase. The dye was measured at $A_{492}$ with a Titertek Multiscan plate reader. Plates containing the same amount of DMSO, but without the test extract were used as controls. The percent cytotoxicity was calculated by comparing the $A_{492}$ readings from the tests relative to $A_{492}$ readings from control wells in which the extract fraction was omitted.

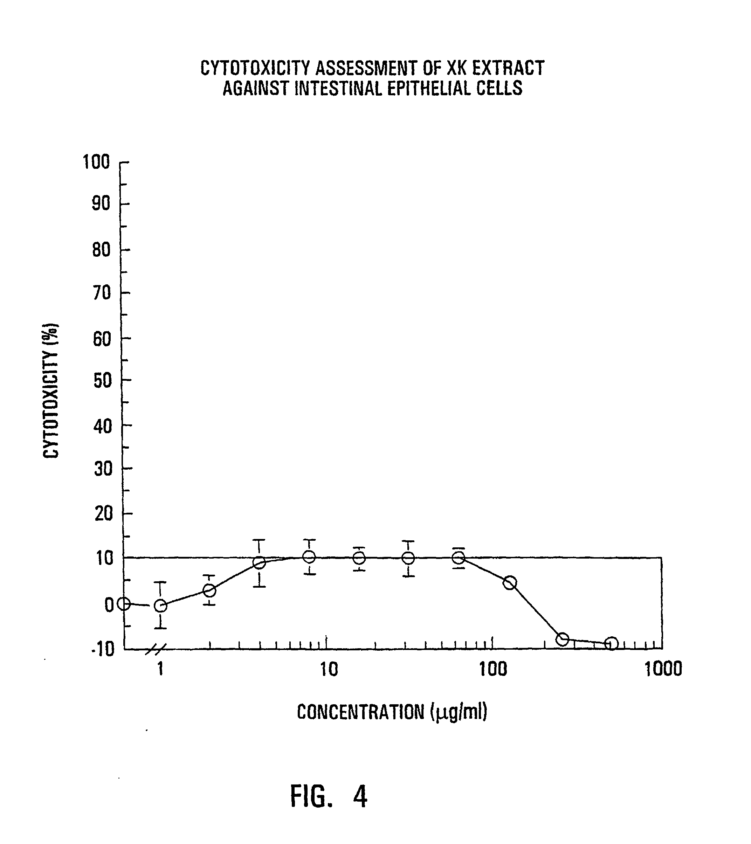

The results of this example confirm that the aqueous extract fraction of *Prunella vulgaris* shows no cytotoxic effect up to the highest concentration tested, i.e., 500 μg/ml (see Figure 4).

The series of experiments described in Examples 1 through 8 above document that invention compositions obtained from *Prunella vulgaris* are polyanionic carbohydrates, which are nontoxic up to 500 μg/ml, and have specific novel activity against enveloped viruses, and specifically, strains of HSV-1, HSV-2, including acyclovir-resistant strains.

The active extract fraction from *Prunella vulgaris* can be purified to homogeneity and its chemical and biological characteristics determined in order to understand its chemical nature, mode of action, and spectrum of activity, and thereby used to improve its utility and potency as an antiviral drug, according to the following examples.

Example 9

Isolation and purification of anti-herpes extract fraction from *Prunella vulgaris*

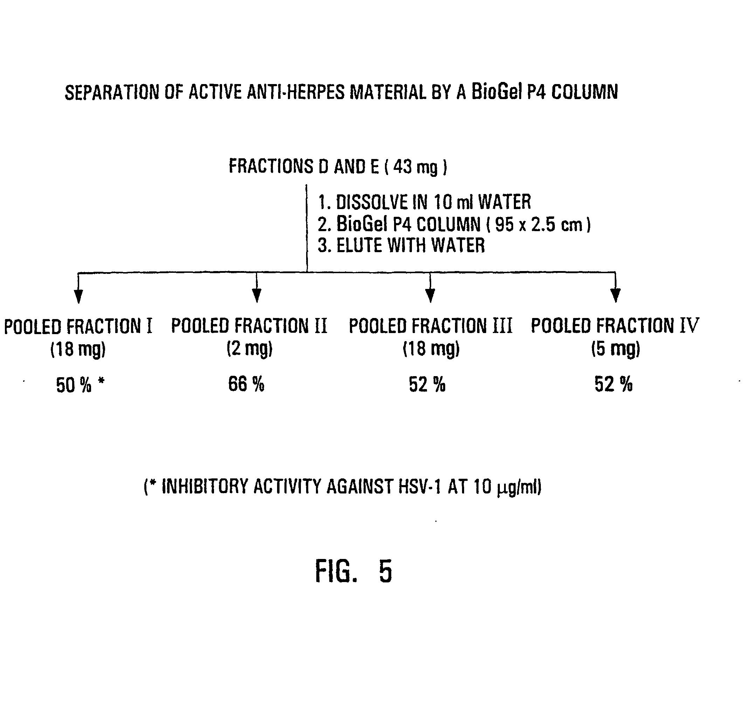

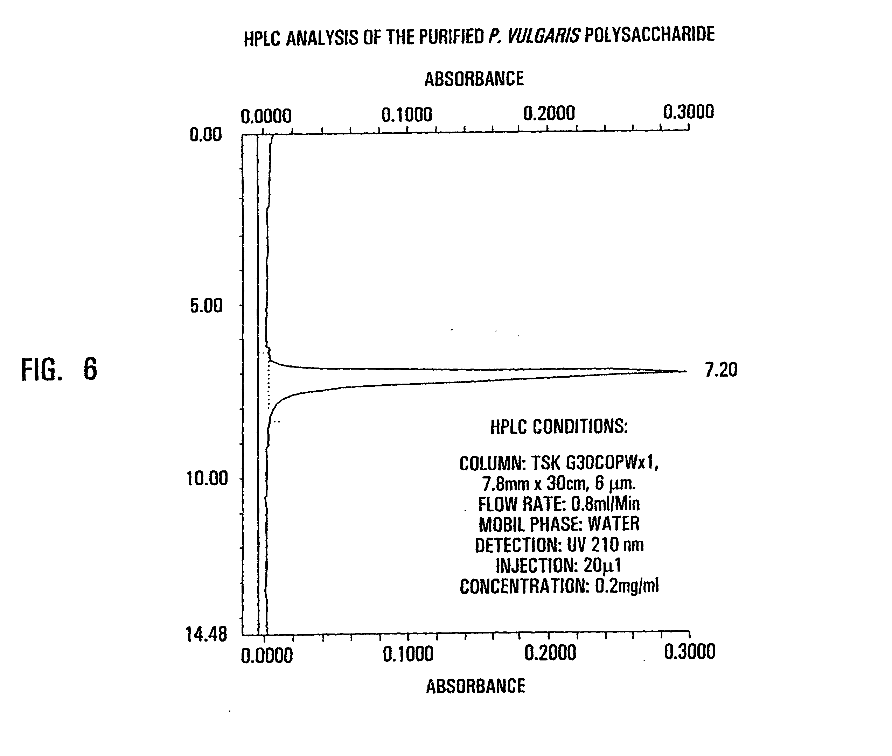

The anti-herpes anionic polysaccharide was purified by rechromatographing pooled materials from Fractions D and E (see Example 2) on a Biogel P4 column (95 x 2.5 cm) using methods as described in Example 2. The yield and inhibitory activity against HSV-1 of fractions obtained are shown in Figure 5. The purified extract fraction of the present invention can also be prepared by extraction, precipitation, and gel filtration chromatography, as disclosed herein. Alternative means for purification, for example, density equilibrium centrifugation, ultrafiltration, and dialysis can also be employed. More economical purification of the active compound for industrial scale processes can be achieved by, for example, ionic interaction chromatography (DEAE-Sepharose 4B) and reversed-phase high pressure liquid chromatography. Since the active compound binds strongly to DEAE Sepharose 4B at neutral pH and can be eluted with 2 M NaCl, the ethanol-precipitated compounds can be applied to a DEAE-Sepharose column, the active compound eluted with a NaCl gradient, and the active fractions identified by standard plaque reduction assays. Thereafter, the active fractions can be pooled, dialyzed, and further purified by HPLC with a preparative reversed-phase (C-18) column, a representative purification process which can scaled up on industrial scale HPLCs. When a sample from fraction IV was analyzed by HPLC, a single peak was obtained, indicating the anti-herpes compound is pure (Fig. 6). The conditions for the HPLC were as follows: column - TSK G3000PWx1, 7.8mm x 30cm, 6μm; flow rate of 0.8 ml/min; mobil phase - water; detection - UV 210nm; injection of 20 μl and a concentration of 0,2 mg/ml.

Example 10

Chemical Characterization of anti-herpes compound from P. vulgaris

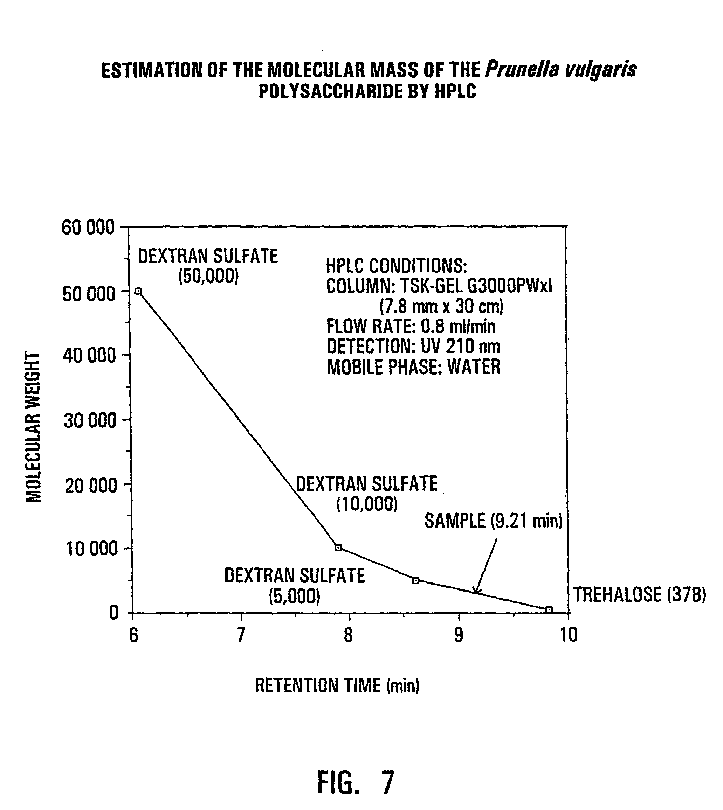

The next objective was to determine the molecular weight or mass of the purified compound. The most commonly employed methods to determine molecular mass of macromolecules are gel permeation chromatography, HPCL exclusion chromatography, osmotic pressure, SDS gel electrophoresis, the Squire method using G-75, dialysis through membranes with selected molecular mass cut-offs, ultracentrifugation with sedimentation rate measurement, and the like. The molecular mass of the purified compound was estimated by HPLC with a get filtration column (TSK-GEL G3000PWxl, 7.8 mm x 30 cm, 6 μm). The purified compound has an estimated molecular mass of 3,500 Da. (Figure 7; This is in contrast to the prunellin previously isolated by Tabba et al.(Antiviral Res. (1989) 11(5-6):263-273) which has a molecular mass of 10,000 Da). Elemental, Infrared, NMR and other spectroscopic analytical means can also be employed to characterize the active compound.

The purified compound was hydrolyzed in 2 N trifluoroacetic acid at 121°C for 1 h. When the hydrolysate was analyzed by paper chromatography (solvent system: pyridine:ethyl acetate:water = 4:10:3), three spots which have the same $R_f$ values of glucose, galactose and xylose standards were obtained. The product can also be exhaustively hydrolyzed in acid (e.g., 2N HCl) and analyzed for constituent monosacharides by HPLC, and gas chromatography. By comparing the intensity of the spots, glucose was the major constituent sugar. Galactose and xylose were minor components.

These results indicate that the purified anti-herpes polysaccharide is composed of mainly glucose with some galactose and xylose as the constituent monosaccharides. Some of these monosaccharides are likely to have $SO_4$ and/ COOH groups on them to confer the anionic nature of the polysaccharide. Known assays, such as uronic acid assay (see Blumenkrantz, N., and G. Asboe-Hansen in Anal. Biocehm. 54:484-489 (1973)) and Molgan-Elson assay (see Ghuysen, J.M., et al., in Methods Enzymology 8:685-699 (1966)), can be employed to verify the amounts of uronic acid and hexosamines present. Other characteristics of the isolated compounds, for example, pI, pH of aqueous solution, and solubility, can be determined by means known in the art.

Example 11

Biological Characterization of anti-herpes compound from P. vulgaris

1. Activity of the purified polysaccahride on HSV

As disclosed herein, the semi-purified extract fraction has two mechanisms of anti-HSV activity *in vitro* by acting extracellularly and intracellularly against the virus. Since the active compound(s) binds extracellularly, thereby reducing the yield of infectious virus and preventing cell-to-cell transmission of the virus, it is likely the compound interferes intracellularly with other functional or biosynthetic steps in the viral replication process.

Specific modes of action can be elucidated by using means known in the art, for example, by the virion binding assay. In this example, $^{35}$S-labeled HSV-1 is purified by sucrose gradient (20-60%, w/w) fractionation of culture fluids from 7-20 h virus-infected Vero cells grown in methionine-deficient medium containing 2% dialyzed fetal bovine serum and $^{35}$S-methionine (10 μCi/ml, specific activity >800 Ci/mmoles, New England Nuclear). The radiolabeled virus is incubated with the purified compound or medium at 4°C and then added to monolayers of Vero cells at a multiplicity of infection (MOI) of 1. Following 1 h incubation at 4°C to allow virus adsorption, cells are washed free of unadsorbed viruses with phosphate-buffered saline containing bovine serum albumin (0.5%). The extent of virus binding can be compared by measuring the radioactivity in monolayers infected with compound-treated or medium-treated virus. Alternatively, monolayer cells can be solubilized with a detergent, and the cell-bound radiolabel analyzed by SDS-PAGE and fluorography.

The major viral capsid protein VP5 can be used as a convenient protein for densitometric quantitation (see Herold, B.C., et al., in J. Virol. 65:1090-1098(1991). Other binding experiments known in the art can be employed to study the effect on invention compound on virus binding kinetics, and on virus already bound to Vero cells.

The specific role of the *Prunella vulgaris* compound in virus binding in relationship to gC and gB can be further validated using gC-deficient and gB-deficient mutants of HSV-1 and specific anti-gC and gB antibodies. The effect of the compound on virus penetration can be studied using known methods in the art, for example, Herold et al. in J. Virol. 70:3461-3469 (1996), which determines the resistance of an adsorbed virus to a pH 3.0 buffer.

Intracellular anti-HSV activity of the compound can be studied by infecting Vero cells with HSV-1 at a MOI of 20 at 4°C, and subsequent virus growth (from extracellular supernatants and cell lysates) in the presence or absence of the compound then compared at 0, 0.5, 1, 2, 4, 8, 12, 16, and 20 h post-infection at 37°C. This example of a "single-cell growth" experiment is expected to confirm the invention results disclosed herein, specifically, that the addition of the compound following infection reduces virus yields in cultures. Ultrastructural studies on these samples can be performed using known methods (see, for example, Gollins and Porterfield in J. Gen. Virol. 66:1969-1982(1985). Comparison of the mode of entry of HSV-1 into cells, with the subsequent morphological development in the cellular and subcellular compartments of infected cells incubated in the presence or absence of the compound, will lead to increased knowledge of the fundamental viral inhibitory characteristics of invention compound and can be used to define the mode of action of invention compound at the biochemical and molecular level.

Table 3

| Activity of the Purified Polysaccharide on HSV | | | | | | |
|---|---|---|---|---|---|---|
| | | | % Plaque Inhibition | | | |
| Virus | Acyclovir sensitivity | Strain #[a] | 50 µg/ml[b] | 25 µg/ml | 12.5 µg/ml | 6.25 µg/ml |
| HSV-1 | Sensitive | 15577 | 100 | 95 | 49 | 0 |
| HSV-1 | Resistant Thymidine kinase deficient | 12959 | 100 | 100 | 14 | 0 |
| HSV-1 | Resistant Thymidine kinase altered | 15518 | 100 | 88 | 20 | 0 |
| HSV-2 | Sensitive | 15614 | 100 | 100 | 41 | 18 |
| HSV-2 | Resistant Thymidine kinase altered | 15597 | 100 | 95 | 49 | 0 |

a Well-characterized strains from Burroughs Wellcome Co.

b Concentrations of the purified anti-herpes compound used.

These results show that the purified polysaccharide from *P. vulgaris* contains activity against both types of herpes viruses regardless of whether they are sensitive or resistant to acyclovir. The results indicate that the *P. vulgaris* compound has a different mode of action than acyclovir and imply that it can used for treatment of infections caused by acyclovir-resistant herpes viruses.

The activity of the purified compound of the extract fraction can be tested against viruses in the herpes family (e.g. VZV and cytomegalovirus) and other enveloped viruses, such as human immunodeficiency virus type 1 (HIV-1), human cytomegalovirus, measles virus, mumps virus, influenza and parainfluenza virus, respiratory syncytial virus, and the like, to determine its full spectrum of activity and anti-coagulant activity, useful for determining its specific utility for a given enveloped virus.

2. Anti-coagulant activity

The anti-coagulant activity of the compound was measured by the prothrombin time test. The prothrombin times were measured at 37°C using a BBL fibrometer (Becton Dickinson and Co, USA). Blood ( 9 ml), collected from the rabbit marginal ear vein, was mixed with 3.8% sodium citrate (1 ml) and centrifuged at 1,500 x g for 10 min at room temperature to obtain a clear supernatant as the testing plasma. In atypical assay, the mixture containing 50 µl of the testing solution (1mg/mL), 150 µl of 50 mM Tris buffer, 0.1 M HCl, pH 7.5, 100 µl of thromboplastin with calcium and 100µL of plasma, was incubated at 37°C. The prothrombin times were recorded in seconds as the fibrometer stopped due to clotting.

The average prothrombin time for the anti-herpes compound was $25.9 \pm 1.5$ seconds, a value similar to the water control $29.9 \pm 1.4$ seconds. Water is employed as a control to show the normal prothrombin time of the plasma. The results showed that our anti-herpes compound has substantially little or no anti-coagulant activity. This is in contrast to a known anti-coagulant anionic polysaccharide, heparin, which has also been described to have anti-HSV activity (Herold et al., (1996) J. Virol. 70:3461-3469. The prothrombin time for anti-coagulant (e.g., heparin) is about 300 seconds.

3. Comparative studies of the anti-herpes activity between the *P. vulgaris* compound and sodium heparin.

The anti-herpes activity of the *P. vulgaris* compound and sodium heparin was studied to illustrate the differences between the two polysaccharides. First, using the plaque reduction assay as described in Example 2, a significant difference in the ability of the two compounds to inhibit HSV-1 strain # 15577 and strain ΔgC2-3 (a glycoprotein C deficient HSV-1, Herold et al., (1994) J. Gen. Virol. 75:1211-1222) was observed. Glycoprotein C is one of the viral envelop proteins that mediates virus binding to heparin receptors on mammalian cells. Heparin is thought to inhibit HSV-1 infection of cells by competing with heparin receptors (Harold et al. (1994)). The $IC_{50}$ of heparin against strains 15577 and ΔgC2-3 were estimated as 750 µg/ml and > 1 mg/ml, respectively. In contrast, the $IC_{50}$ of the *P. vulgaris* compound against strains 15577 and ΔgC2-3 were estimated as 10 µg/ml and 5 µg/ml, respectively.

A second major difference between the *P. vulgaris* polysaccahride and sodium heparin was observed by the binding experiment. In this experiment, 0.1 ml of HSV-1 ($10^5$ pfu) was incubated with 0.1 ml of sodium heparin (20 mg/ml), or the *P. vulgaris* compound (1 mg/ml), or water at 36°C for 1 h. The mixture was serially diluted in medium and the number of residual infectious virus was determined by the plaque assay. The number of plaque developed in the water treatment control was taken as 100%. The results showed that after the treatment with 2 mg of heparin, 30% and 42% of the original HSV-1 strains 15577 and ΔgC2-3, respectively, were still infectious and recovered by the plaque assay. In contrast, the same amount of virus after exposure to 100 μg of the *P. vulgaris* compound, none of the virus from both strains remained infectious and could not be recovered by the plaque assay.

The anti-herpes effect of sodium heparin was further investigated by a binding competition experiment. In this experiment, 0.5 ml samples of HSV-1 (about 100 pfu) were mixed with 0.5 ml of sodium heparin at different concentrations. The mixtures were immediately used to infect Vero cells at 37°C for 1 h. Following the incubation, the number of plaques were determined in the plaque assay. The results showed that there was a 88% inhibition of plaque formation for strain # 15577 when 62.5 μg/ml heparin was used. However, complete inhibition of plaque formation could not be achieved even when 1 mg/ml heparin was used. At 1 mg/ml heparin, the % plaque inhibition was 92%. Similar results were obtained when the gC-deficient HSV-1, ΔgC2-3, was used. In which case, the % plaque inhibition for 62.5 μg/ml and 1 mg/ml heparin were 77% and 86%, respectively.

These results suggest that sodium heparin has anti-herpes activity only when it is present at the start of the virus infection. In other words, heparin can have inhibitory effect on HSV only when the virus has not yet bound to the cells to initiate the infection cycle. This statement is further supported by the high $IC_{50}$ values (750-1000 μg/ml) as determined by the plaque reduction assay. In the plaque reduction assay, heparin was added 1 h after the virus has incubated (i.e. has bound and probably penetrated the cells) with the vero cells. Heparin has no inhibitory effect as indicated by the high $IC_{50}$ values. The anionic polysaccharide heparin exerts its anti-herpes effect by blocking virus binding to the cells. This blocking can only be effective when the virus has not bound to the cell receptors. This is in real contrast to the *P. vulgaris* anionic polysaccharide that it can inhibit HSV infection before virus binding as well as after virus binding and penetration. The results described in the one-step growth study (Example 6) clearly show that even after 7.25 h post virus infection, the *P. vulgaris* polysaccharide could reduce virus yield by 90%. In addition, the results described in this example, specifically in the plaque reduction assay and the binding experiment, clearly show that the P. *vulgaris* polysaccharide is a more superior anti-herpes agent than heparin.

Example 12

*In vivo* Toxicity Testing of Anti-Herpes Compound(s) from *Prunella vulgaris*

The *in vivo* toxicity of invention compound can be tested using means known in the art, for example, a two-step procedure on albino mice as follows:

Step 1: Dose Ranging Determination.

To determine the dose which will produce a toxic effect in mice, the anti-herpes extract PVP dissolved in 0.4 ml of distilled water was administered orally, via a feeding tube, to BALB/c female mice (8 month-old, 22 to 23 gram). A low dose (e.g., 25 mg/kg) of the new compound is administered orally to one animal which is then observed hourly for 24 hours and thereafter, every eight hours, with continuous monitoring daily for 14 days. A second animal receives double the dose of the first animal. The process is then repeated for subsequent animals, each receiving a dose twice that of the previous animal to a maximum of 2000 mg/kg (OCED guidelines 1995). A total of ten animals are used to establish dosing in this step. The amount of PVP administered per animal was 25, 100, 200 400 and 800 mg/kg body weight.

At each of the doses, the animals survived and showed no signs of in a moribund state. A moribund state is characterized by symptoms such as shallow, labored or irregular respiration, muscular weakness or tremors, absence of voluntary response to external stimuli, inability to remain upright, cyanosis and coma. Any one of these indicators will mark a moribund condition, and the animal is euthanized immediately. However, if no moribund effects or lethality were observed, the animals are killed and selected organs (heart, lung, liver, spleen, kidney, brain, muscle, uterus) sampled for histopathology at day 14. Since no moribund effects or lethality was observed, the animals were euthanized and the organs of each animal were examined. Histological examination of the selected organs showed no inflammation or any other pathological signs. These results indicate that PVP has no *in vivo* toxicity at a concentration up to 800 mg/kg.

Step 2: Approximate Acute Toxicity ($LD_{50}$) Determination.

Using dose ranging determinations from Step 1, three appropriate dose levels are established for $LD_{50}$ determination. Ten animals are used for each dose level, and monitored hourly for 24 hours and thereafter, every eight hours, with continuous monitoring daily for 14 days. Animals exhibiting moribund signs are killed immediately; all animals are killed at 14 days and their organs sampled for histology. Using the data from these experiments, actual $LD_{50}$ is estimated by extrapolation and mathematical manipulations using known methods in the art (see, for example De-Pass. in Toxicology Letters 49:159 (1989).

Compounds present in the untreated extract (see Example 2) and in the purified fraction (Example 4) are active in suppressing the cytopathogenic effects of HSV *in vitro.* Based on this latter activity and certain of their biochemical characteristics (particularly their anionic properties) these preparations of the extract are capable of suppressing the cytopathogenicity of other enveloped viruses. This extrapolation is supported by Baba et al (see Antimicrobial Agents and Chemotherapy 32:1742(1988)).

In conclusion, it has been shown that the invention compositions, obtained, for example, from the spikes of *Prunella vulgaris,* is an effective agent for the treatment of the cytopathogenic effects of an enveloped virus in mammals. This result provides insight into the pathway of action of the purified extract fraction within enveloped viruses.

## Claims

1. A composition **characterized** as:

    (1) a water soluble polyanionic polysaccharide comprising glucose, galactose and xylose, wherein glucose is the major constituent, and galactose and xylose are minor components as analyzed by paper chromatography;

    (2) having substantially little or no anti-coagulant activity as measured by the prothrombin time test;

    (3) able to inhibit viral infection before virus binding as well as after virus binding and penetration;

    (4) having substantially little or no *in vivo* toxicity; and

    (5) having a molecular mass when measured by HPLC of approximately 3500 Da.

2. A composition according to claim 1, further **characterized** as:

    (6) having a pH of 5.5 when dispersed in an aqueous solution at a concentration of about 1 mg/ml.

3. A composition according to claims 1 or 2, further **characterized** as:

    (7) having a strong UV absorption peak at 202 nm and a shoulder at 280 nm extending to 380 nm when dispersed in aqueous medium.

4. A composition according to any one of claims 1 to 3, further **characterized** as:

    (8) being stable to exposure to temperatures in the range of about 95-100°C for 4 hours;

    (9) being substantially insoluble in methanol, ethanol, butanol, acetone, and chloroform;

    (10) having an elemental content of about 30.78% carbon, 3.05% hydrogen, 0.66% nitrogen and 2.69% sulfur;

    (11) containing 42% (w/w) carbohydrate, expressed as glucuronic acid;

    (12) containing 7.5% (w/w) uronic acid, expressed as glucuronic acid;

    (13) capable of binding to Alcian blue and to DEAE Sepharose at neutral pH;

    (14) being non-proteinaceous;

    (15) having a retention time of 3.56 min when subjected to reversed-phase high pressure liquid chromatography (HPLC) on a C18 column (25 cm x 4.6 mm ID, 5 μ, Supelcosil LC-18, Sigma) and eluted with 5% water: 95% acetonitrile at a flow rate of 0.3 ml/min; and

(16) being effective for the treatment of the cytopathogenic effects of an enveloped virus in a mammal.

5.  A composition according to any one of claims 1 to 4, wherein the molecular mass is 3500 Da.

6.  A composition according to any one of claims 1 to 5, wherein said composition is **characterized** as being derived from the *Nepetoideae* subfamily of plants.

7.  A composition according to any one of claims 1 to 6, wherein said composition is **characterized** as a compound derived from cells of the plant *Prunella vulgaris.*

8.  A composition according to any one of claims 1 to 6, wherein said composition is obtained from cells of the plant *Prunella vulgaris.*

9.  A pharmaceutical formulation comprising a composition according to any one of claims 1 to 8, and a pharmaceutically acceptable carrier therefor.

10. A method for producing a composition according to any one of claims 1 to 8, wherein said compound is isolated from *Prunella vulgaris* by hot water extraction, ethanol precipitation and gel permeation column chromatography using gel media that permits the purification of a substance having a molecular mass when measured by HPLC of approximately 3500 Da.

11. A composition according to any one of claims 1 to 8 for inhibiting infection of cell(s) by an enveloped virus.

12. A composition according to any one of claims 1 to 8 for treating cytopathogenic effects of an enveloped virus.

13. A composition according to claim 12, wherein cytopathogenic effects of an enveloped virus are acquired as a result of exposure to environmental factors.

14. A composition according to claim 12, wherein the cytopathogenic effects of an enveloped virus are acquired in response to treatment with agents which affect normal immunocompetence.

15. A composition according to any one of claims 11 to 14, wherein said enveloped virus is herpes simplex virus.

16. A composition according to any one of claims 11 to 15, wherein a sample of blood withdrawn from a patient infected with said enveloped virus is incubated with said composition and then transferred back into said patient.

17. A composition according to any one of claim 16, wherein withdrawing, incubating and transferring are repeated at least once.

18. A composition according to any one of claims 11 to 17, for use in a mammal.

19. A composition according to claim 19, wherein said mammal is human.

20. A composition according to any one of claims 11 to 19, wherein said composition is a purified extract fraction analog wherein preferred embodiments of the skeletal structure are that of a polyanionic carbohydrate.

21. A composition according to any one of claims 11 to 20, wherein said composition is for use by continuous infusion, unit dosage, depot or sustained release.

22. A composition according to any one of claims 11 to 21, wherein said composition is for use in a manner that provides biological availability.

23. A composition according to claim 22, wherein said composition is for use parenterally.

24. A composition according to claim 22, wherein said composition is for use topically.

25. A composition according to claim 22, wherein said composition is for use orally.

26. Use of an effective amount of a composition according to any one of claims 1 to 8 for preparing a medicament that inhibits infection of cell(s) by an enveloped virus.

27. Use of an effective amount of a composition according to any one of claims 1 to 8 for preparing a medicament for treating cytopathogenic effects of an enveloped virus.

28. A use according to claim 27, wherein the cytopathogenic effects of an enveloped virus are acquired as a result of exposure to environmental factors.

29. A use according to claim 27, wherein the cytopathogenic effects of an enveloped virus are acquired in response to treatment with agents which affect normal immunocompetence.

30. A use according to any one of claims 26 to 29, wherein said enveloped virus is herpes simplex virus.

31. A use according to any one of claims 26 to 30, wherein a sample of blood withdrawn from a patient infected with said enveloped virus is incubated with said composition and then transferred back into said patient.

32. A use according to claim 31, wherein withdrawing, incubating and transferring are repeated at least once.

33. A use according to any one of claims 26 to 32, wherein the medicament is for use in a mammal.

34. A use according to claim 33, wherein said mammal is human.

35. A use according to any one of claims 26 to 34, wherein said composition is a purified extract fraction analog wherein preferred embodiments of the skeletal structure are that of a polyanionic carbohydrate.

36. A use according to any one of claims 26 to 35, wherein said medicament is for use by continuous infusion, unit dosage, depot or sustained release.

37. A use according to any one of claims 26 to 36, wherein said medicament is for use in a manner that provides biological availability.

38. A use according to claim 37, wherein said medicament is for use parenterally.

39. A use according to claim 37, wherein said medicament is for use topically.

40. A use according to claim 37, wherein said medicament is for use orally.


**Patentansprüche**

1. Zusammensetzung **gekennzeichnet durch**

   **(1)** ein wasserlösliches polyanionisches Polysaccharid, das Glucose, Galactose und Xylose umfasst, worin die Glucose der Hauptbestandteil ist und die Galactose und Xylose geringere Bestandteile, analysiert **durch** Papierchromatographie, sind,

   **(2)** mit im wesentlichen geringer oder gar keiner antikoagulierenden Aktivität, gemessen **durch** den Prothrombin-Zeittest,

   **(3)** die Fähigkeit, virale Infektionen zu hemmen, bevor das Virus bindet, sowie auch nach der Virusbindung und -penetration,

   **(4) durch** im wesentlichen geringe oder gar keine *in vivo*-Toxizität und

   **(5) durch** eine Molekularmasse, gemessen **durch** HPLC, von ungefähr 3500 Da.

2. Zusammensetzung nach Anspruch 1 weiterhin **gekennzeichnet durch**

**(6)** einen pH-Wert von 5,5, wenn sie in einer wässrigen Lösung mit einer Konzentration von etwa 1 mg/ml dispergiert ist.

3. Zusammensetzung nach Anspruch 1 oder 2 weiterhin **gekennzeichnet durch**

**(7)** ein starkes UV-Absorptionspeak bei 202 nm und eine Schulter bei 280 nm, die sich bis zu 380 nm erstreckt, wenn sie in wässrigem Medium dispergiert ist.

4. Zusammensetzung nach einem der Ansprüche 1 bis 3 weiterhin **gekennzeichnet durch**

**(8)** Stabilität gegen Temperaturen im Bereich von etwa 95 bis 100 °C während 4 h,

**(9)** im wesentlichen Unlöslichkeit in Methanol, Ethanol, Butanol, Aceton und Chloroform,

**(10)** einen Elementengehalt von etwa 30,78 % Kohlenstoff, 3,05 % Wasserstoff, 0,66 % Stickstoff und 2,69 % Schwefel,

**(11)** einen Gehalt von 42 % (Gew./Gew.) Kohlenhydrat, ausgedrückt als Glucuronsäure,

**(12)** einen Gehalt von 7,5 % (Gew./Gew.) Uronsäure, ausgedrückt als Glucuronsäure,

**(13)** Fähigkeit zur Bindung an Alcianblau und DEAE-Sepharose bei neutralem pH-Wert,

**(14)** dass sie nicht proteinartig ist,

**(15)** eine Retentionszeit von 3,56 min, wenn man sie einer Umkehrphasen-Hochdruck-Flüssigkeitschromatographie (HPLC) auf einer $C_{18}$-Säule (25 cm x 4,6 mm Innendurchmesser, 5 μ, Supelcosil-LC-18, Sigma) unterzieht und mit 5 % Wasser und 95 % Acetonitril bei einer Fließgeschwindigkeit von 0,3 ml/min eluiert, und

**(16)** Wirksamkeit für die Behandlung der cytopathogenen Effekte eines umhüllten Virus in einem Säugetier.

5. Zusammensetzung nach einem der Ansprüche 1 bis 4, worin die Molekularmasse 3500 Da ist.

6. Zusammensetzung nach einem der Ansprüche 1 bis 5, **dadurch gekennzeichnet, dass** sie sich von der Pflanzenunterfamilie *Nepetoidease* herleitet.

7. Zusammensetzung nach einem der Ansprüche 1 bis 6, worin die Zusammensetzung als eine Verbindung **gekennzeichnet** ist, die sich von Zellen der Pflanze *Prunella vulgaris* herleitet.

8. Zusammensetzung nach einem der Ansprüche 1 bis 6, worin die Zusammensetzung aus Zellen der Pflanze *Prunella vulgaris* erhalten wird.

9. Pharmazeutische Formulierung mit einer Zusammensetzung nach einem der Ansprüche 1 bis 8 und einem pharmazeutisch verträglichen Träger hierfür.

10. Verfahren zur Herstellung einer Zusammensetzung nach einem der Ansprüche 1 bis 8, worin die Verbindung aus *Prunella vulgaris* durch Heißwasserextraktion, Ethanolausfällung und Gelpermeationssäulenchromatographie unter Verwendung von Gelmedium isoliert wird, das die Reinigung einer Substanz mit einer Molekularmasse, gemessen durch HPLC, von ungefähr 3500 Da erlaubt.

11. Zusammensetzung nach einem der Ansprüche 1 bis 8 zur Hemmung von Zell(en)-Infektion durch ein umhülltes Virus.

12. Zusammensetzung nach einem der Ansprüche 1 bis 8 zur Behandlung von cytopathogenen Effekten eines umhüllten Virus.

13. Zusammensetzung nach Anspruch 12, bei der cytopathogene Effekte eines umhüllten Virus als ein Ergebnis dessen, dass man Umgebungsfaktoren aussetzt, erreicht werden.

14. Zusammensetzung nach Anspruch 12, bei der die cytopathogenen Effekte eines umhüllten Virus in Reaktion auf eine Behandlung mit Mitteln erzielt werden, welche normale Immunokonkurrenz beeinflussen.

15. Zusammensetzung nach einem der Ansprüche 11 bis 14, worin das umhüllte Virus *Herpes simplex*-Virus ist.

16. Zusammensetzung nach einem der Ansprüche 11 bis 15, worin eine von einem Patienten, der mit dem umhüllten Virus infiziert ist, abgenommene Blutprobe mit der Zusammensetzung inkubiert und dann zurück in den Patienten überführt wird.

17. Zusammensetzung nach Anspruch 16, wobei das Abnehmen, Inkubieren und Überführen wenigstens einmal wiederholt werden.

18. Zusammensetzung nach einem der Ansprüche 11 bis 17 zur Verwendung in einem Säugetier.

19. Zusammensetzung nach Anspruch 18, worin das Säugetier ein Mensch ist.

20. Zusammensetzung nach einem der Ansprüche 11 bis 19, wobei diese Zusammensetzung eine gereinigte Extraktfraktion analog derjenigen ist, worin bevorzugte Ausführungsformen der Skelettstruktur jene eines polyanionischen Kohlenhydrates sind.

21. Zusammensetzung nach einem der Ansprüche 11 bis 20, bei der die Zusammensetzung für die Verwendung durch kontinuierliche Infusion, Einheitsdosierung, Depot oder verzögerte Wirkstoffabgabe bestimmt ist.

22. Zusammensetzung nach einem der Ansprüche 11 bis 21, bei der diese Zusammensetzung für die Verwendung in einer Weise bestimmt ist, die biologische Verfügbarkeit liefert.

23. Zusammensetzung nach Anspruch 22, bei der die Zusammensetzung für parenterale Verwendung bestimmt ist.

24. Zusammensetzung nach Anspruch 22, worin die Zusammensetzung für örtliche Verwendung bestimmt ist.

25. Zusammensetzung nach Anspruch 22, worin die Zusammensetzung für orale Verwendung bestimmt ist.

26. Verwendung einer wirksamen Menge einer Zusammensetzung nach einem der Ansprüche 1 bis 8 zur Herstellung eines Arzneimittels, das eine Zell(en)-Infektion durch ein umhülltes Virus hemmt.

27. Verwendung einer wirksamen Menge einer Zusammensetzung nach einem der Ansprüche 1 bis 8 zur Herstellung eines Arzneimittels für die Behandlung cytopathogener Effekte eines umhüllten Virus.

28. Verwendung nach Anspruch 27, bei der die cytopathogenen Effekte eines umhüllten Virus als ein Ergebnis dessen, dass man Umgebungsfaktoren aussetzt, erreicht werden.

29. Verwendung nach Anspruch 27, bei der die cytopathogenen Effekte eines umhüllten Virus in Reaktion auf eine Behandlung mit Mitteln erzielt werden, welche normale Immunokonkurrenz beeinflussen.

30. Verwendung nach einem der Ansprüche 26 bis 29, bei der das umhüllte Virus *Herpes implex*-Virus ist.

31. Verwendung nach einem der Ansprüche 26 bis 30, bei der eine von einem Patienten, der mit dem umhüllten Virus infiziert ist, abgenommene Blutprobe mit der Zusammensetzung inkubiert und dann zurück in den Patienten überführt wird.

32. Verwendung nach Anspruch 31, bei der das Abnehmen, Inkubieren und Überführen wenigstens einmal wiederholt werden.

33. Verwendung nach einem der Ansprüche 26 bis 32, bei der das Arzneimittel für die Verwendung in einem Säugetier bestimmt ist.

34. Verwendung nach Anspruch 33, bei der das Säugetier ein Mensch ist.

**35.** Verwendung nach einem der Ansprüche 26 bis 34, bei der die Zusammensetzung eine gereinigte Extraktfraktion analog derer ist, bei der bevorzugte Ausführungsformen der Skelettstruktur jene eines polyanionischen Kohlenhydrates sind.

**36.** Verwendung nach einem der Ansprüche 26 bis 35, bei der das Arzneimittel für die Verwendung durch kontinuierliche Infusion, Einheitsdosierung, Depot oder verzögerte Wirkstoffabgabe bestimmt ist.

**37.** Verwendung nach einem der Ansprüche 26 bis 36, bei der das Arzneimittel für die Verwendung in einer Weise bestimmt ist, die eine biologische Verfügbarkeit liefert.

**38.** Verwendung nach Anspruch 37, bei der das Arzneimittel für parenterale Verwendung bestimmt ist.

**39.** Verwendung nach Anspruch 37, bei der das Arzneimittel für örtliche Verwendung bestimmt ist.

**40.** Verwendung nach Anspruch 37, bei der das Arzneimittel für orale Verwendung bestimmt ist.

**Revendications**

**1.** Composition, **caractérisée par** :

(1) un polysaccharide polyanionique hydrosoluble comprenant du glucose, du galactose et du xylose, le glucose étant le constituant principal, et le galactose et le xylose étant les constituants secondaires suivant l'analyse par chromatographie sur papier ;
(2) une activité anticoagulante très faible ou nulle, mesurée par le test de détermination du temps de prothrombine ;
(3) la capacité à inhiber une infection virale avant la liaison du virus et également après la liaison et la pénétration du virus ;
(4) une toxicité in vivo très faible ou nulle ;
(5) une masse moléculaire, lors de la mesure par CLHP, d'approximativement 3500 Da.

**2.** Composition suivant la revendication 1, **caractérisée en outre par** :

(6) un pH de 5,5 lors de sa dispersion en solution aqueuse à une concentration d'environ 1 mg/ml.

**3.** Composition suivant la revendication 1 ou 2, **caractérisée en outre par** :

(7) un pic intense d'absorption UV à 202 nm et un épaulement à 280 nm s'étendant à 380 nm lors de la dispersion dans un milieu aqueux.

**4.** Composition suivant l'une quelconque des revendications 1 à 3, **caractérisée en outre par** :

(8) la stabilité à l'exposition à des températures comprises dans l'intervalle d'environ 95 à 100°C pendant 4 heures ;
(9) la propriété d'être pratiquement insoluble dans le méthanol, l'éthanol, le butanol, l'acétone et le chloroforme ;
(10) des teneurs en éléments d'environ 30,78 % de carbone, 3,05 % d'hydrogène, 0,66 % d'azote et 2,69 % de soufre ;
(11) la présence de 42 % (en poids/poids) de glucide, exprimé en acide glucuronique ;
(12) la présence de 7,5 % (en poids/poids) d'acide uronique, exprimé en acide glucuronique ;
(13) la capacité de se lier au bleu Alcian et au DEAE-Sepharose à pH neutre ;
(14) une nature non protéique ;
(15) un temps de rétention de 3,56 minutes lorsqu'elle est soumise à une chromatographie en phase liquide sous haute pression (CLHP) à phase inversée sur une colonne C18 (25 cm x 4,6 mm de DI, de 5 μm, Supelcosil LC-18, Sigma) et lorsqu'elle est éluée avec un mélange de 5 % d'eau : 95 % d'acétonitrile à un débit de 0,3 ml/min ; et
(16) l'efficacité pour le traitement des effets cytopathogènes d'un virus à enveloppe chez un mammifère.

**5.** Composition suivant l'une quelconque des revendications 1 à 4, dans laquelle la masse moléculaire est égale à 3500 Da.

**6.** Composition suivant l'une quelconque des revendications 1 à 5, ladite composition était **caractérisée en ce qu'**elle est dérivée de la sous-famille de plantes Nepetoideae.

**7.** Composition suivant l'une quelconque des revendications 1 à 6, ladite composition étant **caractérisée** en tant que composé dérivé de cellules de la plante Prunella vulgaris.

**8.** Composition suivant l'une quelconque des revendications 1 à 6, ladite composition étant obtenue à partir de cellules de la plante Prunella vulgaris.

**9.** Formulation pharmaceutique comprenant une composition suivant l'une quelconque des revendications 1 à 8, et un support pharmaceutiquement acceptable à cette fin.

**10.** Procédé pour la production d'une composition suivant l'une quelconque des revendications 1 à 8, dans lequel ledit composé est isolé de Prunella vulgaris par extraction à l'eau chaude, précipitation par l'éthanol et chromatographie sur colonne de perméation sur gel en utilisant un milieu consistant en un gel qui permet la purification d'une substance ayant une masse moléculaire, lors de la mesure par CLHP, d'approximativement 3500 Da.

**11.** Composition suivant l'une quelconque des revendications 1 à 8 pour inhiber l'infection d'une cellule ou de cellules par un virus à enveloppe.

**12.** Composition suivant l'une quelconque des revendications 1 à 8 pour le traitement des effets cytopathogènes d'un virus à enveloppe.

**13.** Composition suivant la revendication 12, dans laquelle les effets cytopathogènes d'un virus à enveloppe sont acquis en résultat de l'exposition à des facteurs ambiants.

**14.** Composition suivant la revendication 12, dans laquelle les effets cytopathogènes d'un virus à enveloppe sont acquis en réponse à un traitement avec des agents qui affectent l'immunocompétence normale.

**15.** Composition suivant l'une quelconque des revendications 11 à 14, dans laquelle ledit virus à enveloppe est le virus d'herpès simplex.

**16.** Composition suivant l'une quelconque des revendications 11 à 15, dans laquelle un échantillon de sang prélevé chez un patient infecté par ledit virus à enveloppe est mis en incubation avec ladite composition et est ensuite retransféré dans ledit patient.

**17.** Composition suivant l'une quelconque des revendications 16, dans laquelle le prélèvement, l'incubation et le transfert sont répétés au moins une fois.

**18.** Composition suivant l'une quelconque des revendications 11 à 17, destinée à être utilisée dans un mammifère.

**19.** Composition suivant la revendication 19, dans laquelle ledit mammifère est l'homme.

**20.** Composition suivant l'une quelconque des revendications 11 à 19, ladite composition étant un analogue de fraction d'extrait purifié dans lequel les formes préférées de réalisation de la structure de squelette sont celles d'un glucide polyanionique.

**21.** Composition suivant l'une quelconque des revendications 11 à 20, ladite composition étant destinée à être utilisée par perfusion continue, en dose unitaire, sous forme d'un dépôt ou par libération prolongée.

**22.** Composition suivant l'une quelconque des revendications 11 à 21, ladite composition étant destinée à être utilisée de manière à présenter une disponibilité biologique.

**23.** Composition suivant la revendication 22, ladite composition étant destinée à être utilisée par voie parentérale.

**24.** Composition suivant la revendication 22, ladite composition étant destinée à être utilisée par voie topique.

**25.** Composition suivant la revendication 22, ladite composition étant destinée à être utilisée par voie orale.

**26.** Utilisation d'une quantité efficace d'une composition suivant l'une quelconque des revendications 1 à 8 pour la préparation d'un médicament qui inhibe l'infection d'une cellule ou de cellules par un virus à enveloppe.

**27.** Utilisation d'une quantité efficace d'une composition suivant l'une quelconque des revendications 1 à 8 pour la préparation d'un médicament destiné au traitement des effets cytopathogènes d'un virus à enveloppe.

**28.** Utilisation suivant la revendication 27, dans laquelle les effets cytopathogènes d'un virus à enveloppe sont acquis en résultat d'une exposition à des facteurs ambiants.

**29.** Utilisation suivant la revendication 27, dans laquelle les effets cytopathogènes d'un virus à enveloppe sont acquis en réponse au traitement avec des agents qui affectent l'immunocompétence normale.

**30.** Utilisation suivant l'une quelconque des revendications 26 à 29, dans laquelle ledit virus à enveloppe est le virus d'herpès simplex.

**31.** Utilisation suivant l'une quelconque des revendications 26 à 30, dans laquelle un échantillon de sang prélevé chez un patient infecté par ledit virus à enveloppe est mis en incubation avec ladite composition et est ensuite retransféré dans ledit patient.

**32.** Utilisation suivant la revendication 31, dans laquelle le prélèvement, l'incubation et le transfert sont répétés au moins une fois.

**33.** Utilisation suivant l'une quelconque des revendications 26 à 32, dans laquelle le médicament est destiné à être utilisé chez un mammifère.

**34.** Utilisation suivant la revendication 33, dans laquelle ledit mammifère est l'homme.

**35.** Utilisation suivant l'une quelconque des revendications 26 à 34, dans laquelle ladite composition est un analogue de fraction d'extrait purifié dans lequel les formes préférées de réalisation de la structure de squelette sont celles d'un glucide polyanionique.

**36.** Utilisation suivant l'une quelconque des revendications 26 à 35, dans laquelle ledit médicament est destiné à être utilisé par perfusion continue, en dose unitaire, sous forme de dépôt ou par libération prolongée.

**37.** Utilisation suivant l'une quelconque des revendications 26 à 36, dans laquelle ledit médicament est destiné à être utilisé d'une manière assurant la disponibilité biologique.

**38.** Utilisation suivant la revendication 37, dans laquelle ledit médicament est destiné à être utilisé par voie parentérale.

**39.** Utilisation suivant la revendication 37, dans lequel ledit médicament est destiné à être utilisé par voie topique.

**40.** Utilisation suivant la revendication 37, dans laquelle ledit médicament est destiné à être utilisé par voie orale.

**EXTRACTION AND ISOLATION OF ACTIVE ANTI-HERPES MATERIALS FROM THE SPIKES OF *Prunella vulgaris***

*P. vulgaris* (1.4 Kg)

EXTRACT WITH WATER (12 L x 3, 1.5h), 100°C
FILTER THROUGH COTTON

AQUEOUS SOLUTION

FREEZE-DRIED

AQUEOUS EXTRACT ( 85 g. )

AQUEOUS EXTRACT ( 60 g. )

1. DISSOLVE IN WATER (300 ml )
2. ADD EtOH TO 90% (v/v)
3. 4°C OVERNIGHT
4. FILTER WITH COTTON

SUPERNATANT                    PRECIPITATE

OPTIONAL:
[1. EXTRACTED WITH BuOH (1.5 L x 4)]
[2. EXTRACTED WITH MeOH (1.5 L x 3)]

PVP ( 31 g. )

FIG.  1

## SEPARATION OF ACTIVE ANTI-HERPES MATERIAL BY A SEPHADEX G-50 COLUMN

PVP (450 mg)

1. DISSOLVE IN WATER ( 10 ml )
2. SEPHADEX G-50 COLUMN ( 98 x 2.5 CM )
3. ELUTE WITH WATER ( 5 ml OF EACH FRACTION )

| | A | B | C | D | E | F |
|---|---|---|---|---|---|---|
| Fr. 1-40 | Fr. 41-74 | Fr. 75-90 | Fr. 91-104 | Fr. 105-120 | Fr. 121-126 | Fr. 127-150 |
| (0 mg) | (186 mg) | (85 mg) | (58 mg) | (42 mg) | (5 mg) | (32 mg) |
| | 3-20 % * | 6-12 % | 12-48 % | 69-83 % | 85-100 % | 22 % |

(* INHIBITORY ACTIVITY AGAINST HSV-1 AT THE CONCENTRATION OF 25 - 50 $\mu$g/ml)

# FIG. 2

AQUEOUS
EXTRACT

PVP

FRACTION E

FIG. 3A

FIG. 3B

FIG. 3C

CYTOTOXICITY ASSESSMENT OF XK EXTRACT
AGAINST INTESTINAL EPITHELIAL CELLS

FIG. 4

SEPARATION OF ACTIVE ANTI-HERPES MATERIAL BY A BioGel P4 COLUMN

FRACTIONS D AND E ( 43 mg )

1. DISSOLVE IN 10 ml WATER
2. BioGel P4 COLUMN ( 95 x 2.5 cm )
3. ELUTE WITH WATER

| POOLED FRACTION I (18 mg) | POOLED FRACTION II (2 mg) | POOLED FRACTION III (18 mg) | POOLED FRACTION IV (5 mg) |
|---|---|---|---|
| 50 % * | 66 % | 52 % | 52 % |

(* INHIBITORY ACTIVITY AGAINST HSV-1 AT 10 μg/ml)

FIG. 5

HPLC ANALYSIS OF THE PURIFIED *P. VULGARIS* POLYSACCHARIDE

FIG. 6

HPLC CONDITIONS:

COLUMN: TSK G30COPWx1,
7.8mm x 30cm, 6 μm.
FLOW RATE: 0.8ml/Min
MOBIL PHASE: WATER
DETECTION: UV 210 nm
INJECTION: 20μ1
CONCENTRATION: 0.2mg/ml

EP 1 017 727 B1

**ESTIMATION OF THE MOLECULAR MASS OF THE *Prunella vulgaris* POLYSACCHARIDE BY HPLC**

FIG. 7